# EUROPEAN PATENT SPECIFICATION

(11) **EP 2 308 514 B1**
(45) Date of publication and mention of the grant of the patent: **05.06.2013**
(21) Application number: 11152915.2
(22) Date of filing: 21.03.2008
(51) Int. Cl.: A61K 47/48, A61P 31/12

(54) **Conjugates for targeted drug delivery across the blood-brain barrier**
Konjugate zum Arzneimitteltransport über die Blut-Hirn-Schranke
Conjugés pour le transport de médicaments à travers la barrière hémato-encéphalique

(30) Priority: 23.03.2007 US 907176 P
(43) Date of publication of application: 13.04.2011
(62) Divisional of application: 08723917.4
(73) Proprietor: to-BBB Holding B.V., 2333 CH Leiden (NL)
(72) Inventor: Gaillard, Pieter Jaap, 2311 KE Leiden (NL)
(74) Representative: Swinkels, Bart Willem

(56) References cited:
- WO-A2-00/35422
- WO-A2-2004/069870
- US-A1- 2007 141 133
- US-B1- 6 372 250
- NOBLE CHARLES O ET AL: "Development of ligand-targeted liposomes for cancer therapy", EXPERT OPINION ON THERAPEUTIC TARGETS,, vol. 8, no. 4, 1 August 2004 (2004-08-01), pages 335-353, XP009119051, ISSN: 1744-7631, DOI: DOI:10.1517/14728222.8.4.335
- MAEDA N ET AL: "Synthesis of angiogenesis-targeted peptide and hydrophobized polyethylene glycol conjugate", BIOORGANIC & MEDICINAL CHEMISTRY LETTERS, PERGAMON, ELSEVIER SCIENCE, GB, vol. 14, no. 4, 23 February 2004 (2004-02-23), pages 1015-1017, XP002411827, ISSN: 0960-894X, DOI: DOI:10.1016/J.BMCL.2003.11.073
- MARUYAMA K ET AL: "CONSTRUCTION OF TRANSFERRIN-PEG-LIPOSOMES FOR INTRACELLULAR DRUG DELIVERY IN SOLID TUMOR IN VIVO", JOURNAL OF LIPOSOME RESEARCH, TAYLOR & FRANCIS, PHILADELPHIA, PA, US, vol. 8, no. 1, 1 January 1998 (1998-01-01) , page 81/82, XP002941048, ISSN: 0898-2104
- MORE S S ET AL: "Design, synthesis and biological evaluation of glutathione peptidomimetics as components of anti-Parkinson prodrugs", JOURNAL OF MEDICINAL CHEMISTRY, AMERICAN CHEMICAL SOCIETY, US, vol. 51, no. 15, 14 August 2008 (2008-08-14), pages 4581-4588, XP002602082, ISSN: 0022-2623, DOI: 10.1021/JM800239V [retrieved on 2008-07-24]

## Description

### Field of the invention

This invention relates to the field of targeted drug delivery. The invention relates to conjugates that are obtainable by reacting distearoylphosphatidylethanolamine-polyethylene glycol-maleimide (DSPE-PEG-MAL) with reduced glutathione. These conjugates are used for preparing medicaments for targeted drug delivery of drugs into and across the blood-brain barrier.

### Background of the invention

A virus is a microscopic particle that can infect the cells of an organism. Viruses can only replicate by infecting a host cell and therefore cannot reproduce on their own. At the most basic level, viruses consist of genetic material, DNA and/or RNA, contained within a protective protein coat called a capsid. To enter a cell, a virus attaches to the cell surface via binding to a specific receptor. Subsequently, the virus is taken up by the cell either by means of direct cell membrane fusion (using cell penetrating peptides), or by via an endocytotic vesicle. Virusses use the machinery and metabolism of the host cell to produce multiple copies of themselves via a lytic and/or a lysogenic cycle. Released virions can be passed between hosts either through direct contact, often via body fluids, or through a vector. In aqueous environments, viruses float free in the water.

Currently, there are limited options in the treatment of viral conditions. Antiviral therapies may either target the virus before it enters the cell, as is the case in both passive immunization (i.e., antibody therapies) and active immunization or vaccinations, or it may interfere with the intracellular uptake and/or replication cycles of the virus, whereby type I interferons, and inducers thereof, increase the natural antiviral mechanisms of the body.

For antiviral drugs to reach their intracellular targets, they need to be delivered across the lipophilic cell membrane, into the hydrophilic cytoplasm. This lipophilic to hydrophilic transport requirement forms a challenge for the design and delivery of such antiviral drugs.

The currently effective and marketed antiviral drugs that belong to the class of intracellularly active compounds, like nucleoside analogs, protease inhibitors and nucleic acid based drugs, are hydrophilic drugs and therefore usually have very poor intracellular uptake, but generally have good pharmacokinetic and safety properties, allowing very high systemic exposure to the drug. In some cases, these drugs are selectively accumulated via endogenous uptake carriers, such as e.g., nucleoside or anion transporters, in cells and organs with high expression of these uptake carriers. This may, however, prevent the antiviral drugs to reach effective concentrations in the intracellular compartment of target cells, *i.e.,* cells infected with virus, and/or present dose-limiting toxicity in the cells and organs with high expression of these uptake carriers.

Increasing the lipophilicity of the drugs will result in a relatively higher partitioning to lipid-rich membranes and also an increased affinity for drug-inducible multidrug efflux pumps, both leading to reduced and variable cytosolic concentrations of the drug as well as an increased likeliness of systemic dose limiting toxicity for the drug.

Administering drugs in a non-phosphorylated form, may improve cell entrance of a drug, since the cell membrane is poorly permeable to phosphorylated drugs. Subsequently, the drug may be phosphorylated into its active form by a thymidine kinase. However, drug-uptake will occur non-specifically by all body tissues, including the central nervous system (CNS). Another drawback is that, it may take up to 4 weeks of dosing to achieve steady state plasma levels of the drug. Current treatments require daily administration of high doses (800-1200 mg/day) for periods of 24-48 weeks. This is too late for the treatment of non-chronic conditions, such as (sub)acute virus-induced diseases. Yet another drawback is that such treatments are usually limited by toxicity, with the most frequent side effects being the development of hemolytic anemia or kidney damage, requiring either dose reduction or discontinuation in certain patients, with a consequent reduction in response to therapy. In fact, the percentage of patients who achieve a sustained viral response using such drugs is usually at best 50-60%, even though these drugs were effective for the particular virus using in vitro assays.

In conclusion, in antiviral treatment and therapy there is a need for the delivery of an effective amount of drugs in a suitable time period to a desired site while minimizing side effects. Perhaps the biggest challenge lies in the timely delivery of an antiviral drug to sites protected by physiological barriers, such as the central nervous system (CNS), the retina and the testes.

There are still significant unmet medical needs for the rapidly growing and mostly under treated population of (ageing) patients that suffer from severely disabling or life-threatening neurological or central nervous system (CNS) disorders, like viral encephalitis. One particular reason is that most drugs have difficulties crossing the brain's firewall, the blood-brain barrier. The brain is the most complex and sensitive organ of the human body. It requires a delicate ion and neurotransmitter balance around neurons to function properly, and many endogenous and exogenous potential neurotoxic compounds are constantly threatening the homeostasis of the brain. Just like a firewall protects a computer from potentially harmful intruders from the Internet, physical and functional barriers within the blood vessels of the brain serve to protect neurons. These barriers do so by excluding, effluxing and metabolizing potential neurotoxic compounds (including plasma proteins, cytokines, antibodies, drugs, bacteria and viruses) from blood and brain. This so-called blood-brain barrier, or BBB, is characterized by a uniquely specialized tight endothelial cell layer that covers the smallest blood vessels (capillaries) in the brain. For the uptake of essential nutrients, however, the blood-brain barrier is equipped with specific carrier systems and uptake receptors. Just like a computer' firewall has dedicated ports for communication with the Internet. Since almost every neuron is perfused by its own capillary, the most effective way of delivering drugs to the brain, would be achieved through these capillaries. In fact, the total length of capillaries in the human brain is impressive (-600 km) with a large surface area (-20 m2) for effective exchange of drugs. Currently marketed CNS drugs are therefore usually either very small and potent water soluble compounds (-300 Da) or highly lipid soluble compounds, so that these compounds can cross the blood-brain barrier by diffusion. Major limitations of strong lipophilic drugs are, however, that such compounds have poor drug-like properties, are usually strong substrates for drug efflux transporters and present dose-limiting toxicities within their therapeutic range. Alternatively, marketed small molecule drugs mimic endogenous substrates for uptake carriers (e.g., the Parkinson drug L-dopa uses an amino acid carrier to cross the blood-brain barrier). There are no CNS drugs on the market yet that target specific uptake receptors. A large portion of the marketed drugs for the treatment of neurological disorders (like stroke, migraine and MS), are in fact directed against targets outside the brain (e.g., cerebral vasculature, or immune system). Unlike small molecules, biopharmaceutical drugs are unlikely candidates for chemical modifications to enhance their permeability across the blood-brain barrier. Such compounds now rely on invasive and harmful technologies to patients, like direct and local stereotactic injections, intrathecal infusions and even (pharmacological) disruption of the blood-brain barrier. Because of the severe neurological consequences of these techniques, these are only warranted in selected life-threatening diseases. Moreover, local administrations are far from effective in delivering drugs throughout the large human brain. Innovative CNS drug delivery technologies are thus highly awaited.

Still, despite considerable efforts, these approaches have thus far not delivered many new safe CNS drugs. Briefly summarized, the first comprise the currently marketed 'natural' CNS active drugs are likely substitutes for additional CNS indications, where, based on advanced medicinal chemistry, many screening libraries of CNS penetrating compounds have been built (in silico) that may provide new CNS drugs. Secondly, many cell penetrating vectors are being developed based on viral vectors and peptide vectors. Thirdly, blood-brain barrier circumventing technologies, like stereotactic injections, ICV/intrathecal infusion pumps, encapsulated drug producing cells, nasal administration and inhalers, are being explored to (locally) deliver (biopharmaceutical) drugs into the brain. Fourthly, brain absorption enhancing technologies by breaching the blood-brain barrier for small molecules, including RMP-7, osmotic disruption and drug efflux pump (P-glycoprotein) inhibitors, have all been extensively investigated in clinical trials and, though effective, abandoned by the companies due to safety concerns. Finally, chemical modifications / formulations of potential CNS drugs (like synthesis of blood-brain barrier penetrating pro-drugs or lipidification of drugs), are strategies to enhance brain delivery of small molecule CNS drugs. Such technologies principally only alter the distribution of the drug throughout the whole body, which then results in a moderately larger brain uptake as well. This increases the chance of peripheral side effects. Unlike small molecules, biopharmaceutical drugs are unlikely candidates for chemical modifications to enhance their permeability across the blood-brain barrier, with the exception of cationisation. An increased adsorptive-mediated endocytosis, the mechanism of action used by peptide vectors and cationisation, may however result in neurotoxic side effects as this goes against the neuroprotective nature of the blood-brain barrier. Others technologies are local or harmful to patients and are therefore only allowed to be applied in selected life-threatening diseases. Although selected CNS indications will certainly benefit from these approaches, the specific and widespread delivery of drugs across the blood-brain barrier could best be achieved by targeting to endogenous internalizing uptake (transport) receptors on the capillaries in the brain, without disrupting the neuroprotective blood-brain barrier.

Currently there are no drugs on the market yet that employ CNS drug targeting technologies. Targeted small molecule CNS drugs are being developing based on endogenous substrates for uptake carriers (like used by L-dopa). Such uptake carriers, however, allow very little chemical modifications to the endogenous substrates, making this approach only suitable for a small and unpredictable number of potential CNS drugs.

It is an object of the invention to provide for a safe, effective and versatile approach for carrying cargo such as large proteins and liposomes containing drugs and genes across the cell membrane and across a blood-tissue barrier such as the blood-brain barrier for *inter αliα* CNS drug targeting.

### Description of the invention

### Definitions

The terms "oligonucleotide" and "polynucleotide" as used herein include linear oligo- and polymers of natural or modified monomers or linkages, including deoxyribonucleosides, ribonucleosides, α-anomeric forms thereof, polyamide nucleic acids, and the like, capable of specifically binding to a target polynucleotide by way of a regular pattern of monomer-to-monomer interactions (e.g. nucleoside-to-nucleoside), such as Watson-Crick type of base pairing, Hoogsteen or reverse Hoogsteen types of base pairing, or the like. Usually monomers are linked by phosphodiester bonds or analogs thereof to form oligonucleotides ranging in size from a few monomeric units, e.g. 3-4, to several hundreds of monomeric units. Whenever an oligonucleotide is represented by a sequence of letters, such as "ATGCCTG," it will be understood that the nucleotides are in 3' order from left to right and that "A" denotes deoxyadenosine, "C" denotes deoxycytidine, "G" denotes deoxyguanosine, and "T" denotes thymidine, unless otherwise noted. Analogs of phosphodiester linkages include phosphorothioate, phosphorodithioate, phosphoroselenoate, phosphorodiselenoate, phosphoroanilothioate, phosphoranilidate, phosphoramidate, N3'→P5' phosphoramidate and the like. A polynucleotide can be of substantially any length, typically from about 10 nucleotide to about 1x10⁹ nucleotide or larger. As used herein, an "oligonucleotide" is defined as a polynucleotide of from 4 to 100 nucleotide in length. Thus, an oligonucleotide is a subset of polynucleotides.

As used herein, the term "specific binding" means binding that is measurably different from a non-specific interaction. Specific binding can be measured, for example, by determining binding of a molecule (ligand) compared to binding of a control molecule (ligand), which generally is a molecule of similar structure that does not have binding activity, for example, a peptide of similar size that lacks a specific binding sequence. Specific binding is present if a ligand has measurably higher affinity for the receptor than the control ligand. Specificity of binding can be determined, for example, by competition with a control ligand that is known to bind to a target. The term "specific binding," as used herein, includes both low and high affinity specific binding. Specific binding can be exhibited, e.g., by a low affinity targeting agent having a Kd of at least about 10⁻⁴ M. E.g., if a receptor has more than one binding site for a ligand, a ligand having low affinity can be useful for targeting the microvascular endothelium. Specific binding also can be exhibited by a high affinity ligands, e.g. a ligand having a Kd of at least about of 10⁻⁷ M, at least about 10⁻⁸ M, at least about 10⁻⁹ M, at least about 10⁻¹⁰ M, or can have a Kd of at least about 10⁻¹¹ M or 10⁻¹² M or greater. Both low and high affinity-targeting ligands are useful for incorporation in the conjugates of the present invention.

### Detailed description of the invention

The present invention is based on this mechanism a safe, endogenous (non-toxic) transport mechanism, called receptor-mediated endocytosis, for carrying therapeutic moieties, such as large proteins and liposomes containing drugs and genes across a cell membrane or across a blood-tissue barrier such as the blood-brain barrier for e.g. brain delivery thereof. A range of validated and well known internalizing receptors are present at cells and the blood-brain barrier for the use of the embodiments of the current invention. These include, but are not limited to, the thiamine transporter, alpha(2,3)-sialoglycoprotein receptor, transferrin receptor, scavenger receptors, LDL receptors, LRP1B, LRP2, DTR, insulin receptor, IGF receptor, leptin receptor, mannose 6-phosphate receptor. The present invention thus relates to a safe and effective way of specifically delivering drugs to cells and across the blood-brain barrier by targeting to endogenous internalizing uptake (transport) receptors on the capillaries in the brain, without disrupting the neuroprotective blood-brain barrier.

In a first aspect the present invention relates to a conjugate that is obtainable by reacting distearoylphosphatidylethanolamine-polyethylene glycol-maleimide (DSPE-PEG-MAL) with reduced glutathione.

A "conjugate" is herein defined as consisting of two entities that are coupled together. Preferably, the two entities are conjugated by non-specific or specific protein-protein interaction, by covalent bonding, by non-covalent bonding or by coordinating chemical bonding. In the context of the present invention the first entity may be an antiviral agent or a pharmaceutically acceptable carrier comprising the antiviral agent as herein defined below, whereas the second entity will usually be a ligand for a receptor on a target cell as herein defined below.

### Antiviral agents

The conjugates of the invention may comprise at least one antiviral agent. An "antiviral agent" (or antiviral compound or drug) herein defined as an agent that kills viruses or suppresses their replication and, hence, inhibits their capability to multiply and reproduce. In a preferred conjugate of the invention, the antiviral agent is an intracellularly active antiviral agent. An "intracellularly active antiviral agent" is herein understood to mean an agent that acts to inhibit viral infection and preferably inhibits viral replication inside a virally infected cell, as opposed to neutralizing antibodies that are capable of neutralizing virions circulating extracellularly. Usually an intracellularly active antiviral agent intereferes with an essential step in the virus' replicative metabolism.

A preferred intracellularly active antiviral agent for incorporation in the conjugates of the invention is chemical antiviral agent. A chemical antiviral agent is herein understood to be a defined chemical molecule, usually a smaller, non-polymeric molecule (e.g. less than 2kDa) that is at least partially organic, that usually may be obtained by chemical synthesis and that does not comprise an oligo- or poly-nucleotide. A preferred chemical antiviral agent for incorporation in the conjugates of the invention is an agent that is at least one of a nucleoside analogue, a reverse transcriptase inhibitor, a protease inhibitor, and a neuraminidase inhibitor. Suitable examples of chemical antiviral agents for incorporation in the conjugates of the invention include e.g. the nucleotide reverse transcriptase inhibitor (NtRTI) tenofovir disoproxil fumarate; the non-nucleoside reverse transcriptase inhibitors (NNRTIs) nevirapine, delavirdine and efavirenz; the protease inhibitors saquinavir, ritonavir, indinavir, nelfinavir, amprenavir, lopinavir, darunavir and atazanavir; the neuraminidase inhibitors peramivir, zanamivir (Tamiflu) and oseltamivir (Relenza); amantadine and rimantadine; and adefovir dipivoxil, famciclovir, penciclovir, imiquimod, docosanole, foscarnet (PFA), maribavir, BAY 38-4766, GW275175X, MVE-1, MVE-2, AM-3, AM-5, mannozym, bropirimine, 3,6-bis(2-p-peridinoethoxy) acridine trihydrochloride, phenyleneamine, 2-amino-5-halo-6-aryl-4(3H)-pyrimidinones, 2-amino-5-bromo-6-methyl-4(3H)-pyrimidinone, 7,8-didehydro-7-methyl-8-thioxoguanosine, 7-deazaguanosine, melatonin, 8-chloro-7-deazaguanosine, CL246,738, glycyrrhizin, pleconaril, bananin, iodobananin, vanillinbananin, ansabananin, eubananin, adeninobananin, cloroquine, valinomycin, and compounds as detailed in WO2006119646, WO2005107742, EP1736478, EP1707571, WO2004062676, EP1674104, WO2006060774, W02006121767.

Suitable antiviral nucleoside analogues for incorporation in the conjugates of the invention include nucleoside analogues having an altered sugar, base or both. Examples of suitable nucleoside analogues include e.g. idoxuridine, aciclovir (acyclovir or acycloguansoine), valaciclovir (valacyclovir), ganciclovir, valganciclovir, adenosine arabinoside (AraA, Vidarabine), AraA monophosphate, cytosine arabinoside (AraC, cytarabine), cytosine arabinoside monophosphate (Ara-CMP), azidothymidine (AZT), 1-beta-D-ribofuranosyl-1,2,4-triazole-3-carboxamide (ribavirin or RBV), 5-ethynyl-1-beta-D-ribofuranosylimidazole-4-carboxamide (EICAR), EICAR- monophosphate, ribamidine, ribavirin 2',3',5'-acetate, ribavirin-5'-sulfamate, ribavirin 5'-triphosphate, ribavirin 5'-monophosphate, ZX-2401, mycophenolic acid, tiazofurin, tiazofurin-5'-monophosphate, tiazofurin 2',3',5'-acetate, 7-thia-8-oxoguanosine, selenazofurin, pyrazofurin, furanonaphthoquinone derivatives, merimepodib (VX497), viramidine, 6-azauridine, 9-(2-phosphonylmethoxyethyl)guanine (PMEG), (S)-9-(3-hydroxy-2-phosphonylmethoxypropyl)adenine (HPMPA), 9-(2-phosphonylmethoxyethyl)adenine (PMEA), 9-(2-phosphonylmethoxyethyl)-2,6-diaminopurine (PMEDAP), didenosine (DDI), dideoxycytosine (DDC), stavudine (d4T), Epivir (3TC), abacavir (ABC), iododeozyuridine (DU), and bromovinyl deoxiuridine (BVDU or brivudin), (S)-1-(3-hydroxy-2-phosphonylmethoxypropyl)cytosine (HPMPC, cidofovir, CDV or Vistide®), cyclic HPMPC, hexadecyloxypropyl-cidofovir (HDP-CDV, or CMX001), 3-deazaguanine (3-DG), 3-deazauridine, 9-(S)-(2,3-dihydroxypropyl)adenine ((S)-DHPA), zidovudine, didanosine, zalcitabine, stavudine, lamivudine, abacavir and emtricitabine.

In an alternative embodiment of the conjugates of the invention, the antiviral agent is an agent comprising an oligo- or poly-nucleotide. An antiviral agent comprising an oligo- or poly-nucleotide may be any one of a DNA vaccine, an antisense oligonucleotide, a ribozyme, a catalytic DNA (DNAzyme) or RNA molecule, an siRNA or an expression construct encoding therefor. A DNA vaccine is herein understood to mean an nucleic acid construct comprising a sequence encoding a viral antigen, that is capable of expressing the antigen upon introduction of the construct into a cell of a host organism that is to be vaccinated with the DNA vaccine.

Suitable intracellularly active antiviral agents comprising oligo- or polynucleotides include e.g. DNA vaccines (US20050163804), antisense oligonucleotides (ISIS 13312, ISIS 2922 (fomivirsen), ISIS 3383, ISIS 5320, GEM 132), ribozymes, catalytic DNA (DNAzymes) or RNA molecules (as detailed in, but not limited to WO2006064519, WO2005085442), siRNAs or plasmids encoding thereof (as e.g. detailed in, but not limited to, WO2006042418, WO2006041290, WO2006074346, WO2006062596, WO2006110688, WO2005056021, WO2005076999, WO2006121464, WO2005019410; WO03079757, WO2006096018, WO2006129961, WO2006031901, WO02081494, WO2005028650, WO03070750), or combinations thereof and the like.

Preferred antiviral agents for use in accordance with the invention are agents that are specific or have a sufficient specificity to virally infected cells while displaying minimum toxicity for non-infected host cells. This specificity for infected cells of the agent may be expressed as the 'selectivity index', which is herein defined as the 50% cytostatic concentration (CC50) to the target cell divided by the 50% effective concentration (EC50) that is toxic to a virus. A selectivity index that favors the use of the antiviral is desirable. In a preferred embodiment, the selectivity index is from about 1 to about 100000, more preferably from about 10 to about 100000, even more preferably from about 100 to about 100000, most preferably from about 1000 to about 100000. The selectivity index is thus preferably at least about 1, 10, 100, 1000, 10000 or 100000.

In a preferred embodiment the antiviral agent that is used in accordance with the invention is at least one of ribavirin, cidofovir, ganciclovir, aciclovir, zanamivir and oseltamivir.

### Ribavirin

Ribavirin (originally also known as Virazole; Copegus®; Rebetol®; Ribasphere®; Vilona®, Virazole®, also generics from Sandoz, Teva, Warrick) is a synthetic chemical not found in nature. It was first synthesized in 1970 at ICN Pharmaceuticals, Inc. (later Valeant Pharmaceuticals International). Ribavirin was discovered as part of a systematic ICN search of antiviral and anti-tumor activity in synthetic nucleosides. This was inspired in part by discovery (in the 1960's) of antiviral activity from naturally-occurring purine-like nucleoside antibiotics like showdomycin, coformycin, and pyrazomycin. These agents had too much toxicity to be clinically useful (and the antiviral activity of them may be incidental), but they served as the starting point for pharmaceutical chemists interested in antivirals and anti-metabolic chemotherapeutic agents. In 1972 it was reported that ribavirin was active against a variety of RNA and DNA viruses in culture and in animals, without undue toxicity. Ribavirin protected mice against mortality from both A and B strains of influenza, and ICN originally planned to market it as an anti-influenza drug. Results in human trials against experimental influenza infection were mixed, however, and the FDA ultimately did not approve this indication for ribavirin use in humans. Although ICN was allowed in 1980 to market ribavirin, in inhalant form, for respiratory syncytial virus (RSV) infection in children, the U.S. market for this indication was small. By the time oral ribavirin was finally approved by the FDA as part of a combination treatment (with interferon) for hepatitis C in 1998, the original ICN patents on ribavirin itself had expired, and (notwithstanding subsequent patent disputes) ribavirin had become essentially a generic drug.

Ribavirin is an antiviral drug which is active against a number of DNA and RNA viruses. It is a member of the nucleoside anti-metabolite drugs that interfere with duplication of viral genetic material. Though not effective against all viruses, ribavirin is remarkable as a small molecule for its wide range of activity, including important activities against influenzas, flaviviruses and agents of many viral hemorrhagic fevers. Ribavirin is a pro-drug, activated by cellular kinases which change it into the 5' triphosphate nucleotide. In this form it interferes with aspects of RNA metabolism related to viral reproduction. Without wishing to be bound to any theory, a number of mechanisms have been proposed for this, but none of these is proven. More than one mechanism may be active. In the U.S., the oral (capsule or tablet) form of ribavirin is used in the treatment of hepatitis C, in combination with interferon drugs. The aerosol form is used to treat respiratory syncytial virus-related diseases in children. In Mexico, ribavirin ("ribavirina") has been sold for use against influenza.

The primary serious adverse effect of ribavirin is hemolytic anemia, which may worsen preexisting cardiac disease. This effect is dose-dependent and may sometimes be compensated by decreasing dose, however the mechanism for the adverse effect is unknown. Ribavirin is not substantially incorporated into DNA, but does have a dose-dependent inhibiting effect on DNA synthesis, as well as having other effects on gene-expression. Without wishing to be bound to any theory, these may be the reasons that significant teratogenic effects have been noted in all non-primate animal species on which ribavirin has been tested. Ribavirin did not produce birth defects in baboons, but this should not be an indication that it is safe in humans. Therefore, two simultaneous forms of birth control are recommended during treatment of either partner and continued for six months after treatment. Women who are pregnant or planning to become pregnant are advised not to take ribavirin. Of special concern as regards teratogenicity is the ribavirin's long half-life in the body. Red blood cells (erythrocytes) concentrate the drug and are unable to excrete it, so this pool is not completely eliminated until all red cells have turned over, a process estimated to take as long as 6 months. Thus in theory, ribavirin might remain a reproductive hazard for as long as 6 months after a course of the drug has ended. Drug packaging information materials in the U.S. now reflect this warning.

Experimental data indicate that ribavirin may have useful activity against many viruses of interest, including (avian) influenza, hepatitis B, polio, measles and smallpox. Ribavirin is the only known treatment for a variety of viral hemorrhagic fevers, including Ebola virus, Marburg virus, Lassa fever, Crimean-Congo hemorrhagic fever, and Hantavirus infection. Ribavirin is active in a hamster model of yellow fever, a finding which is not surprising, given the familial relationship of yellow fever and hepatitis C viruses as flaviviridae. Ribavirin is active against other important flaviviridae such as West Nile virus and dengue fever.

Ribavirin's carboxamide group can resemble adenine or guanosine, depending on its rotation, and for this reason when ribavirin is incorporated into RNA, it pairs equally well with either cytosine or uridine, inducing mutations in RNA-dependent replication in RNA viruses. Such hypermutation can be lethal to RNA viruses. Ribavirin 5' mono-di- and tri-phosphates, in addition, are all inhibitors of certain viral RNA-dependent RNA polymerases which are a feature of RNA viruses (except for retroviruses). Neither of these mechanisms explains ribavirin's effect on many DNA viruses, which is more of a mystery. Ribavirin 5'-monophosphate inhibits cellular inosine monophosphate dehydrogenase, thereby depleting intracellular pools of GTP. Without wishing to be bound to any theory, this mechanism may be useful in explaining the drug's general cytotoxic and anti-DNA replication effect (i.e. its toxicity) as well as some effect on DNA viral replication. Ribavirin is an inhibitor of some viral RNA guanylyl transferase and (guanine-7N-)-methyl transferase enzymes, and this may contribute to a defective 5'-cap structure of viral mRNA transcripts and therefore inefficient viral translation for certain DNA viruses, such as vaccinia virus (a complex DNA virus). It has been suggested that incorporation of ribavirin into the 5' end of mRNA transcripts would mimic the 7-methyl guanosine endcap of cellular mRNAs, causing poor cellular translation of these. This would be a cell-toxic effect, but it does not seem to be important at therapeutic ribavirin concentrations. Any difference between cellular and viral enzyme handling of ribavirin-containin mRNA transcripts, is a potential mechanism of differential inhibition of ribavirin to translation of mRNAs from viruses (including DNA viruses). Finally, ribavirin is known to enhance host T-cell-mediated immunity against viral infection through helping to switch the host T-cell phenotype from type 2 to type 1. This may explain rivavirin's antiviral activity against some viruses such as hepatitis C, at doses which do not clearly interfere with replication of the virus when used without interferon.

Ribavirin is absorbed from the GI tract probably by nucleoside transporters. Absorption is about 45%, and this is modestly increased (to about 75%) by a fatty meal. Once in the plasma, ribavirin is transported through the cell membrane also by nucleoside transporters. After several weeks of daily administrations, ribavirin is widely distributed in all tissues, including the CSF and brain. The pharmacokinetics of ribavirin is dominated by trapping of the phosphated form inside cells, particularly red blood cells (RBCs) which lack the enzyme to remove the phosphate once it has been added by kinases, and therefore attain high concentrations of the drug. Most of the kinase activity which converts the drug to active nucleotide form, is provided by adenine kinase. This enzyme is more active in virally infected cells. The volume of distribution of ribavirin is large (2000 L/kg) and the length of time the drug is trapped varies greatly from tissue to tissue. The mean half-life for multiple doses in the body is about 12 days (30 minutes after single dose), but very long-term kinetics are dominated by the kinetics of RBCs (half-life 40 days). RBCs store ribavirin for the lifetime of the cells, releasing it into the body's systems when old cells are degraded in the spleen. About a third of absorbed ribavirin is excreted into the urine unchanged, and the rest is excreted into urine as the de-ribosylated base 1,2,4-triazole 3-carboxamide, and the oxidation product of this, 1,2,4- triazole 3-carboxylic acid.

### Cidofovir

Cidofovir (HPMPC or Vistide) is an acyclic nucleoside phosphonate with broad-spectrum activity against a wide variety of DNA viruses including herpesviruses (Herpes simplex virus type 1 (HSV-1) and type 2 (HSV-2), varicella-zoster virus (VZV), cytomegalovirus (CMV), Epstein-Barr virus (EBV), human herpesvirus type 6 (HHV-6) and equine and bovine herpesviruses), papovaviruses (human polyoma virus and human papilloma virus (HPV)), adeno-, irido-, hepadna-, and poxviruses. Cidofovir has proved effective against these viruses in different cell culture systems and/or animal models. The mechanism of action of Cidofovir is based upon the interaction of its active intracellular metabolite, the diphosphorylated HPMPC derivative HPMPCpp, with the viral DNA polymerase. HPMPCpp has been shown to block CMV DNA synthesis by DNA chain termination following incorporation of two consecutive HPMPC molecules at the 3'-end of the DNA chain.

Cidofovir confers a prolonged antiviral action, which lasts for several days or weeks, thus allowing infrequent dosing (i.e. every week or every two weeks). This prolonged antiviral action is probably due to the very long intracellular half-life of the HPMPC metabolites, particularly the HPMPCp-choline adduct. In clinical studies, Cidofovir has proved efficacious in the treatment of CMV retinitis, following both intravenous injection (3 or 5 mg/kg, every other week) and intravitreal injection (single dose of 20 micrograms per eye). Initial clinical trials also point to the efficacy of both systemic (intravenous) and topical Cidofovir (1% ointment) in the treatment of acyclovir-resistant HSV infections, and of topical Cidofovir (ointment or injection) in the treatment of pharyngeal, laryngeal and anogenital HPV infections. Cidofovir is now being pursued in the topical and/or systemic (intravenous) treatment of various infections due to CMV, HSV, VZV, EBV, HPV, polyoma-, adeno- and poxviruses.

Interestingly, Cidofovir is the only currently marketed antiviral drug shown to be active against JC Virus, the causative virus for PML in immune compromised patients, including the recently reported patients treated with immunosupressive drugs like Tysabri and Rituxan. The major side effect of cidofovir is that it can be nephrotoxic. Cidofovir has also shown efficacy in the treatment of acyclovir resistant herpes. Cidofovir might have anti-smallpox efficacy and might be used on a limited basis in the event of a bioterror incident involving smallpox cases.

### Ganciclovir

Ganciclovir (GCV) was the first antiviral agent approved for treatment of CMV disease, and remains the first-line treatment for CMV infection and CMV disease in transplant recipients. GCV is an acyclic nucleoside analogue of 2'-deoxyguanosine. In a multi-step process dependent on both viral and cellular enzymes, ganciclovir is converted to ganciclovir triphosphate, the chemical form that is active against CMV. The initial phosphorylation is catalyzed by an unusual protein kinase homolog encoded by the CMV UL97 open reading frame. Cellular enzymes generate the triphosphate form. Ganciclovir triphosphate competitively inhibits DNA synthesis catalyzed by the viral DNA polymerase (encoded by the UL54 gene), with slower chain elongation resulting from incorporation of ganciclovir triphosphate in place of dGTP into the growing viral DNA chain.

Absorption of the oral form is very limited - about 5% fasting, about 8% with food. It achieves a concentration in the central nervous system of about 50% of the plasma level. About 90% of plasma ganciclovir is eliminated unchanged in the urine, with a half-life of 2-6 hrs, depending on renal function (elimination takes over 24 hours in end-stage renal disease). GCV as an intravenous (IV) formulation (Cytovene-IV®, Roche) was approved in 1989 for treatment of CMV retinitis in AIDS patients. The IV formulation was later approved for prevention of CMV disease in solid organ transplants (SOT) recipients and in individuals with advanced HIV infection at risk for CMV disease.

The side effects of GCV include serious hematologic adverse effects (common adverse drug reactions (≥1% of patients) include: granulocytopenia, neutropenia, anemia, thrombocytopenia, fever, nausea, vomiting, dyspepsia, diarrhoea, abdominal pain, flatulence, anorexia, raised liver enzymes, headache, confusion, hallucination, seizures, pain and phlebitis at injection site (due to high pH), sweating, rash, itch, increased serum creatinine and blood urea concentrations) and, based on preclinical toxicologic studies, probable long-term reproductive toxicity. In animal studies GCV was both carcinogenic and teratogenic and caused aspermatogenesis.

To circumvent the risks and inconvenience associated with the need for an indwelling catheter for intravenous administration, an oral formulation was developed. Oral GCV (250 and 500 mg GCV capsules; Cytovene®, Roche) was approved in 1994 for treatment of CMV retinitis, but only as maintenance therapy, as the low bioavailability (approximately 5%) of the oral formulation was considered insufficient for induction therapy. Oral GCV represented a major advance in treatment options for maintenance therapy and prophylaxis. However, the low bioavailability and the high pill burden from the t.i.d. regimen were limitations. In addition, there were concerns that inadequate viral suppression resulting from the lower systemic exposure from oral GCV could lead to emergence of drug resistance. Development of prodrugs has been one valuable strategy in circumventing problems of poor solubility or low bioavailability.

To date (2007), no anti-CMV agent has been approved for treatment of CMV induced encephalitis. However, while the oral GCV formulation would avoid the considerable risks and disadvantages associated with IV administration, the hematologic and reproductive toxicities would remain, limiting the usefulness of the therapy for all but the most severely affected.

### Aciclovir

Aciclovir or acyclovir is a guanine analogue antiviral drug primarily used for the treatment of herpes simplex virus infection. It is one of the most commonly-used antiviral drugs, and is marketed under trade names such as Zovirax and Zovir (GSK). Aciclovir was seen as the start of a new era in antiviral therapy, as it is extremely selective and low in cytotoxicity. Acyclovir is an analogue of 2'-deoxyguanosine. Like GCV, acyclovir must be phosphorylated in a multi-step process in the host cell to the active triphosphate form. Aciclovir differs from previous nucleoside analogues in that it contains only a partial nucleoside structure ― the sugar ring is replaced by an open-chain structure. It is selectively converted into a monophosphate form by viral thymidine kinase, which is far more effective (3000 times) in phosphorylation than cellular thymidine kinase. Subsequently, the monophosphate form is further phosphorylated into the active triphosphate form, aciclo-GTP, by cellular kinases. Aciclo-GTP is a very potent inhibitor of viral DNA polymerase; it has approximately 100 times higher affinity to viral than cellular polymerase. Its monophosphate form also incorporates into the viral DNA, resulting in chain termination. It has also been shown that the viral enzymes cannot remove aciclo-GMP from the chain, which results in inhibition of further activity of DNA polymerase. Aciclo-GTP is fairly rapidly metabolized within the cell, possibly by cellular phosphatases. Therefore, aciclovir can be considered a prodrug ― it is administered in an inactive (or less active form) and is metabolized into a more active species after administration.

Aciclovir is active against most species in the herpesvirus family. In descending order of activity: Herpes simplex virus type I (HSV-1), Herpes simplex virus type II (HSV-2), Varicella zoster virus (VZV), Epstein-Barr virus (EBV), Cytomegalovirus (CMV). Activity is predominately active against HSV, and to a lesser extent VZV. It is only of limited efficacy against EBV and CMV. It is inactive against latent viruses in nerve ganglia. The CMV-encoded protein kinase pUL97 catalyzes the initial phosphorylation step of this purine analog, which, like GCV monophosphate, is subsequently di- and tri-phosphorylated by host kinases. ACV is a less efficient substrate than GCV, which in part explains the lower in vitro potency of ACV compared to GCV in CMV-infected cells. Another factor that clearly differentiates ACV and GCV is the four- to five-fold shorter half-life of ACV-TP compared to GCV-TP in infected cells, resulting in the lower intracellular levels of the active ACV-TP. As with GCV, drug resistance to ACV results from mutations in the viral DNA polymerase or UL97 genes.

Aciclovir is poorly water soluble and has poor oral bioavailability (10―20%), hence intravenous administration is necessary if high concentrations are required. When orally administered, peak plasma concentration occurs after 1―2 hours. Aciclovir has a high distribution rate, only 30% is protein-bound in plasma. The elimination half-life of aciclovir is approximately 3 hours. It is renally excreted, partly by glomerular filtration and partly by tubular secretion.

Common adverse drug reactions ≥1% of patients) associated with systemic aciclovir therapy (oral or IV) include: nausea, vomiting, diarrhoea and/or headache. In high doses, hallucinations have been reported. Infrequent adverse effects (0.1―1 % of patients) include: agitation, vertigo, confusion, dizziness, edema, arthralgia, sore throat, constipation, abdominal pain, rash and/or weakness. Rare adverse effects (<0.1% of patients) include: coma, seizures, neutropenia, leukopenia, crystalluria, anorexia, fatigue, hepatitis, Stevens-Johnson syndrome, toxic epidermal necrolysis and/or anaphylaxis. Additional common adverse effects, when aciclovir is administered IV, include encephalopathy (1% of patients) and injection site reactions. The injection formulation is alkaline (pH 11), and extravasation may cause local tissue pain and irritation. Renal impairment has been reported when aciclovir is given in large, fast doses intravenously, due to the crystallization of aciclovir in the kidneys. Since aciclovir can be incorporated also into the cellular DNA, it is a chromosome mutagen, therefore, its use should be avoided during pregnancy. However it has not been shown to cause any teratogenic nor carcinogenic effects. The acute toxicity (LD50) of aciclovir when given orally is greater than 1 mg/kg, due to the low oral bioavailability. Single cases have been reported, where extremely high (up to 80 mg/kg) doses have been accidentally given intravenously without causing any major adverse effects.

### Zanamivir (Relenza)

Zanamivir (5-acetamido-4-guanidino-6-(1,2,3-trihydroxypropyl)-5,6-dihydro-4H-pyran-2-carboxylic acid) is a neuraminidase inhibitor used in the treatment of and prophylaxis of both Influenzavirus A and Influenzavirus B. Zanamivir was the first neuraminidase inhibitor commercially developed.

Bioavailability is 2% (oral) and protein binding <10%. Excretion is renal with negligible metabolism and a half life of 2.5―5.1 hours. Whilst zanamivir proved to be a potent and effective inhibitor of influenza neuraminidase and inhibitor of influenza virus replication in vitro and in vivo, this didn't necessarily translate into a successful clinical treatment for influenza. In clinical trials it was found that zanamivir was able to reduce the time to symptom resolution by 1.5 days provided therapy was started within 48 hours of the onset of symptoms. A further limitation concerns the poor oral bioavailability of zanamivir. This meant that oral dosing was impossible, limiting dosing to the parenteral routes. Zanamivir, therefore, is administered by inhalation - a route that was chosen for patient compliance with therapy. But even this route of administration is not acceptable to many in the community.

Zanamivir was the first of the neuraminidase inhibitors. Despite the limited commercial success of this drug, the work and strategies employed in the development of zanamivir were important first-steps in the development of further members of this class including oseltamivir and the candidate drug RWJ-270201 (Phase I trials). As a result more effective and potent treatments for influenza may be developed in the future.

### Oseltamivir (Tamiflu)

Oseltamivir ((3R,4R,5S)-4-acetylamino-5-amino-3-(1-ethylpropoxy)-1-cyclohexene-1-carboxylic acid ethyl ester), is an antiviral drug that is used in the treatment and prophylaxis of both Influenzavirus A and Influenzavirus B. Like zanamivir, oseltamivir is a neuraminidase inhibitor. It acts as a transition-state analogue inhibitor of influenza neuraminidase, preventing new viruses from emerging from infected cells. Oseltamivir also appears to be active against canine parvovirus, feline panleukopenia, the canine respiratory complex known as "kennel cough," and the emerging disease dubbed "canine flu", an equine virus that began affecting dogs in 2005. Veterinary investigation of its use for canine parvo and canine flu is ongoing, but many shelters and rescue groups have reported great success employing oseltamivir in the early stages of these illnesses.

Oseltamivir was the first orally active neuraminidase inhibitor commercially developed. It is a prodrug, which is hydrolyzed hepatically to the active metabolite, the free carboxylate of oseltamivir (GS4071). Bioavailability is 75% (oral) and excretion of GS4071 is renal with hepatic metabolism to GS4071 and a half life of 6―10 hours. Oseltamivir is indicated for the treatment of infections due to influenza A and B virus in people at least one year of age, and prevention of influenza in people at least 1 year or older. The usual adult dosage for treatment of influenza is 75 mg twice daily for 5 days, beginning within 2 days of the appearance of symptoms and with decreased doses for children and patients with renal impairment. Oseltamivir may be given as a preventive measure either during a community outbreak or following close contact with an infected individual. Standard prophylactic dosage is 75 mg once daily for patients aged 13 and older, which has been shown to be safe and effective for up to six weeks. It has also been found that the standard recommended dose incompletely suppresses viral replication in at least some patients with H5N1 avian influenza, rendering therapy ineffective and increasing the risk of viral resistance. Accordingly, it has been suggested that higher doses and longer durations of therapy should be used for treatment of patients with the H5N1 virus. It has been suggested that co-administration of oseltamivir with probenecid could extend the limited supply of oseltamivir. Probenecid reduces renal excretion of the active metabolite of oseltamivir. One study showed that 500 mg of probenecid given every six hours doubled both the peak plasma concentration (Cmax) and the half-life of oseltamivir, increasing overall systemic exposure (AUC) by 2.5-fold.

Common adverse drug reactions (ADRs) associated with oseltamivir therapy include: nausea, vomiting, diarrhea, abdominal pain, and headache. Rare ADRs include: hepatitis and elevated liver enzymes, rash, allergic reactions including anaphylaxis, and Stevens-Johnson syndrome. Various other ADRs have been reported in postmarketing surveillance including: toxic epidermal necrolysis, cardiac arrhythmia, seizure, confusion, aggravation of diabetes, and hemorrhagic colitis. In May 2004, the safety division of Japan's health ministry ordered changes to the literature accompanying oseltamivir to add neurological and psychological disorders as possible adverse effects, including: impaired consciousness, abnormal behavior, and hallucinations. Various cases of psychological disorders were alleged to be associated with oseltamivir therapy between 2000―2004, including several deaths. On 2005-11-18 the United States Food and Drug Administration (FDA) issued a report regarding the pediatric safety of oseltamivir, which stated that there was insufficient evidence to claim a causal link between oseltamivir use and the deaths of 12 Japanese children (only two from neurological problems). However, it was recommended that a warning was added to the Product Information regarding rashes associated with oseltamivir therapy. In November 2006, reports of bizarre behavior in Japanese children, including three deaths from falls, resulted in the FDA amending the warning label to include possible side effects of delirium, hallucinations, or other related behavior.

### Ligands

The second entity in the conjugates is a ligand for a receptor on a target cell, whereby the receptor is a receptor that mediates at least one of endocytosis and transcytosis (of the ligand). Receptor-mediated delivery is one possible targeted drug delivery technique that was developed in recent years. It has the potential advantage of high specificity of delivery to the cells which express a receptor for the ligand that is conjugated with a drug or a drug carrier. The specific targeting of low molecular weight, as well as polypeptide and nucleic-acid based therapeutic antiviral agents to cells and tissues may be enhanced greatly through the use of receptor-mediated delivery. Moreover, uptake receptors that are also expressed on endothelial cells of the blood-brain barrier and brain parenchymal cells (neurons and neuroglia), will allow the specific delivery of such targeted antiviral agents to the central nervous system (CNS) for the treatment of e.g., viral encephalitis. Receptor-mediated targeting may further be combined with non-specific drug delivery systems (like protein conjugates, PEGylation, nanoparticles, liposomes, and the like) to greatly improve the pharmacokinetic and biodistribution properties of the drugs, which will significantly redirect the drugs specifically to receptor-expressing cells, tissues and organs, including the ones protected by specific blood-tissue barriers like e.g., the CNS, the blood-brain barrier (BBB), the retina and the testes.

In a preferred embodiment therefore, the ligand that is to be incorporated in the conjugates of the invention, is a ligand for an endogenous receptor on a target cell. The ligand preferably is a ligand for a receptor of a vertebrate target cell, more preferably a receptor of a mammalian target cell, and most preferably a receptor of a human target cell. The ligand preferably is a ligand that specifically binds to the receptor. Specific binding of a ligand to a receptor preferably is as defined herein above.

A wide array of uptake receptors and carriers, with a even wider number of receptor-specific ligands, are known in the art. Preferred ligands for receptors that mediates endocytosis and/or transcytosis for use in accordance with present invention include e.g. ligands for, or that specifically bind to the thiamine transporter, folate receptor, vitamin B12 receptors, asialoglycoprotein receptors, alpha(2,3)-sialoglycoprotein receptor (with e.g., the FC5 and FC44 nanobodies consisting of llama single-domain antibodies (sdAbs) as receptor-specific ligands), transferrin-1 and -2 receptors, scavenger receptors (class A or B, types I, II or III, or CD36 or CD163), low-density lipoprotein (LDL) receptor, LDL-related protein 1 receptor (LRP1, type B), the LRP2 receptor (also known as megalin or glycoprotein 330), diphtheria toxin receptor (DTR, which is the membrane-bound precursor of heparin-binding epidermal growth factor-like growth factor (HB-EGF)), insulin receptor, insulin-like growth factors (IGF) receptors, leptin receptors, substance P receptor, glutathione receptor, glutamate receptors and mannose 6-phosphate receptor.

Preferred ligands that bind to these receptors, include e.g. ligands selected from the group consisting of: lipoprotein lipase (LPL), α2-macroglobulin (α2M), receptor associated protein (RAP), lactoferrin, desmoteplase, tissue- and urokinase-type plasminogen activator (tPA/uPA), plasminogen activator inhibitor (PAI-1), tPA/uPA:PAI-1 complexes, melanotransferrin (or P97), thrombospondin 1 and 2, hepatic lipase, factor VIIa/tissue-factor pathway inhibitor (TFPI), factor Villa, factor IXa, Aβ1-40, amyloid-β precursor protein (APP), C1 inhibitor, complement C3, apolipoproteinE (apoE), pseudomonas exotoxin A, CRM66, HIV-1 Tat protein, rhinovirus, matrix metalloproteinase 9 (MMP-9), MMP-13 (collagenase-3), spingolipid activator protein (SAP), pregnancy zone protein, antithrombin III, heparin cofactor II, α1-antitrypsin, heat shock protein 96 (HSP-96), platelet-derived growth factor (PDGF), apolipoproteinJ (apoJ, or clusterin), Aβbound to apoJ and apoE, aprotinin, angio-pep1, very-low-density lipoprotein (VLDL), transferrin, insulin, leptin, an insulin-like growth factor, epidermal growth factors, lectins, peptidomimetic and/or humanized monoclonal antibodies or peptides specific for said receptors (e.g., sequences HAIYPRH and THRPPMWSPVWP that bind to the human transferrin receptor, or anti-human transferrin receptor (TfR) monoclonal antibody A24), hemoglobin, non-toxic portion of a diphtheria toxin polypeptide chain, all or a portion of the diphtheria toxin B chain (including DTB-His (as described by Spilsberg et al., 2005, Toxicon., 46(8):900-6)), all or a portion of a non-toxic mutant of diphtheria toxin CRM197, apolipoprotein B, apolipoprotein E (e.g., after binding to polysorb-80 coating on nanoparticles), vitamin D-binding protein, vitamin A/retinol-binding protein, vitamin B12/cobalamin plasma carrier protein, glutathione and transcobalamin-B 12.

In one preferred embodiment, the ligand is one of a large number of ligands that are shared between the LRP1 and LRP2 receptors, including e.g. lipoprotein lipase (LPL), α2-macroglobulin (α2M), receptor associated protein (RAP), lactoferrin, desmoteplase, tissue- and urokinase-type plasminogen activator (tPA/uPA), plasminogen activator inhibitor (PAI-1), and tPA/uPA:PAI-1 complexes.

In another preferred embodiment , the ligand is more specific for the LRP1 receptor, including, but not limited to, melanotransferrin (or P97), thrombospondin 1 and 2, hepatic lipase, factor VIIa/tissue-factor pathway inhibitor (TFPI), factor Villa, factor IXa, Aβ1-40, amyloid-β precursor protein (APP), C1 inhibitor, complement C3, apolipoproteinE (apoE), pseudomonas exotoxin A, CRM66, HIV-1 Tat protein, rhinovirus, matrix metalloproteinase 9 (MMP-9), MMP-13 (collagenase-3), spingolipid activator protein (SAP), pregnancy zone protein, antithrombin III, heparin cofactor II, α1-antitrypsin, heat shock protein 96 (HSP-96), and platelet-derived growth factor (PDGF, mainly involved in signaling) (154-156), whereas apolipoproteinJ (apoJ, or clusterin), Aβ bound to apoJ and apoE, aprotinin, angio-pep1, and very-low-density lipoprotein (VLDL) are more specific for the LRP2 receptor.

Other preferred ligands are transferrin, insulin, leptin, an insulin-like growth factor, epidermal growth factors, lectins, peptidomimetic and/or humanized monoclonal antibodies or peptides specific for said receptors (e.g., sequences HAIYPRH and THRPPMWSPVWP that bind to the human transferrin receptor, or anti-human transferrin receptor (TfR) monoclonal antibody A24), hemoglobin, non-toxic portion of a diphtheria toxin polypeptide chain, all or a portion of the diphtheria toxin B chain (including DTB-His), all or a portion of a non-toxic mutant of diphtheria toxin CRM197, apolipoprotein B, apolipoprotein E (e.g., after binding to polysorb-80 coating on nanoparticles), glutathione, vitamin D-binding protein, vitamin A/retinol-binding protein, vitamin B12/cobalamin plasma carrier protein, or transcobalamin-B12.

### Carriers

The ligands in the conjugates of the invention may be conjugated to a pharmaceutically acceptable carrier that comprises the antiviral agents. In such conjugates, the antiviral agents may e.g. be encapsulated within nanocontainers, such as nanoparticles, liposomes or nanogels, whereby the ligand is preferably conjugated coupled to such a nanocontainer. Such conjugation to the nanocontainer may be either directly or via any of the well-known polymeric conjugation agents such as sphingomyelin, polyethylene glycol (PEG) or other organic polymers. Details of producing such pharmaceutical compositions comprising targeted (PEG) liposomes are described in US Patent No.6,372,250. Thus, in a preferred embodiment a conjugate according to invention is a conjugate wherein the pharmaceutically acceptable carrier comprises at least one of: a carrier protein, a nanocontainer, a liposome, a polyplex system, a lipoplex system, and, polyethyleneglycol.

In conjugates wherein the antiviral agent comprises a poly- or oligonucleotide, the pharmaceutically acceptable carrier preferably is a lipoplex system comprising at least one of cationic lipids or amphoteric lipids (as detailed in WO2002/066012), or a polyplex system comprising at least one of poly-L-Lysine, poly-L-ornithine, polyethyleneimine, and polyamidoamine. There are two major kinds of non-viral delivery systems for the intracellular delivery of nucleic acid based antiviral drugs (like DNA vaccines, antisense oligonucleotides, ribozymes, catalytic DNA (DNAzymes) or RNA molecules, siRNAs or plasmids encoding thereof), comprising lipoplex systems (cationic liposomes containing DNA) and polyplex systems (DNA attached to a cationic polymer). In a preferred embodiment, the pharmaceutical acceptable carrier is a lipoplex system or a polyplex system. In addition, the pharmaceutical acceptable carrier may further preferably comprise a protein conjugate, polyethyleneglycol (PEGylation), a nanoparticle or a liposome. Polyplex systems comprise cationic polymers such as poly-L-Lysine (PLL), poly-L-ornithine (POL), polyethyleneimine (PEI), polyamidoamine (PAM) or combinations thereof with DNA. Polycationic systems enter cells mainly by adsorptive or fluid-phase endocytosis. Cationic polymers, including PEI, have the ability to condens DNA and to destabilize the membrane potential. Moreover, it has been shown that plasmid delivery by PEI polyplex systems could be achieved by controlling the physical chemical and biological properties of the complex. However, transfection efficiency and gene expression are limited compared to viral transduction systems. Since PEI systems may perturb membranes they can cause also toxicity which correlates with the molecular weight and the nuclear concentration of the polymer. In this respect it was shown that linear PEI (22 kDa) was more toxic than branched PEI (25 kDa) and also related to the amount of PEI used in polyplex systems as expressed by the N/P ration (amount of nitrogen in the polymer related to the amount of DNA). Others state that linear PEI polyplex systems exhibited improved cell viability and a higher transfection efficiency. Recently various biodegradable PEI-derivatives have been synthesized with better transfection properties and less toxicity than linear PEI.

Overall, the efficacy of PEI and probably of polycationic systems in general depends on the molecular weight, the overall cationic charge and the degree of branching. When attached to DNA, other factors like the amount of DNA, the particle size and the zeta potential are important features. Furthermore, the positively charged polycationic systems interact readily with the negatively charged plasma proteins when administered intravenously and opsonization occurs following binding to blood proteins which target them to be cleared by the reticulo-endothelial system (RES). Particulary, the formation of aggregates leads to the uptake by phagocytic cells and the entrapment by capillary networks (mainly lungs following intravenous administration) which results in a fast clearance from the plasma compartment and a poor transfection of target tissues/organs. However, this can be dramatically reduced by PEGylation. Furthermore, application of a targeting/internalization ligand avoids the need to apply polyplex systems with a large N/P ratio and therefore a high overall positive charge and may therefore reduce many problems that are associated with cationic polymers (such as toxicity, binding to blood constituents).

Naked lipoplex systems are also readily opsonized by serum components and cleared by similar mechanisms as polyplex systems e.g. by the reticulo-endothelial system (RES). Moreover, although the unmethylated CpGs of lipoplex systems are masked preventing an innate immune response, once they are in the general circulation, lipoplex systems may be, similarly like polyplex systems, opsonized by blood proteins (C3, IgG, lipoproteins and fibronectin) resulting in inflammatory reactions (mediated by TNF-alpha, IL-6 and IL-12) in lungs and liver. In addition, complex activation and activation of T-, B-, NK-cells and macrophages has been found and were related to the injected dose of the lipoplex. Next to reducing the number of unmethylated CpGs, such interactions can be limited by PEGylation of these systems or by using immunosuppressive agents (e.g. dexamethasone). In addition, the kinetics of these systems are considerably improved by PEGylation reducing their systemic clearance and increasing targeting efficiency (by application of selective/specific targeting ligands). Moreover, decreasing the size of lipoplex systems seems to be a key factor in their tissue distribution and cellular uptake and increases their transfection efficiency. Generally, the tissue distribution and the persistence of expression of lipoplex and polyplex systems is mainly dependent, like with small molecular drugs following parenteral administration, also on the pharmacokinetics (clearance, distribution volume), the formulation (size, charge, PEGylation, etc.) and the dosage regimen (high volume bolus, sequential injection, constant infusion). With respect to dosage regimen it is interesting to note that sequential injection of lipoplexes and plasmid DNA resulted in higher expression but also minimized cytokine induction. Furthermore, the delivery to the target tissue/organ is depending on blood flow and tissue/organ uptake or permeability and the balance of clearances of target and non-target tissues/organs. Therefore a proper dosage regimen, based on pharmacokinetic parameters, should be designed to optimize delivery/targeting to organs and tissues. In addition, this should be harmonized with respect to the intracellular pharmacokinetics.

The intracellular pharmacokinetics (distribution, elimination) of lipoplex and polyplex systems following cellular uptake is an important issue. Apart from receptor-mediated uptake, the internalization (via the clathrin- or the caveolae-dependent route) of particularly untargeted PEI-systems seem to depend on both cell line and the PEI-polyplex type (linear PEI vs branched PEI). Frequently, such systems end up in late endosomes therefore they need to escape from these organelles to enter the cytoplasm to ultimately reach the nucleus. Cationic systems like polyplex systems can escape from the endosomes/lysosomes because they have the ability to buffer pH and cause osmotic swelling of these organelles according to the so-called "proton sponge-mediated escape" theory. Nevertheless, it seems that a small fraction of the internalized systems escapes into the cytoplasm and that a large part stays in the endosomes/lysosomes and is degraded. However, it has been shown that incorporation of fusogenic lipids or cationic peptides (mellitin) into these systems could enhance their endosomal escape.

Once in the cytosol linear plasmids can be readily degraded by nucleases while circular plasmids are much more stable. Circular (desoxy)nucleic acid molecules are therefore preferred. Particularly calcium sensitive nucleases seem to be responsible for this degradation. Ultimately the plasmids have to be transported into the nucleus via the nuclear pore complex (NPC) which forms an aqueous channel through the nuclear envelope and it was estimated that about 0.1 % of plasmids are able to enter the nucleus from the cytosol. Molecules smaller than 40 kDa can passively pass the NPC while larger molecules (> 60 kDa) need a specific nuclear localization signal (NLS) to be actively transported through the NPC permitting transport of molecules up to 25-50 MDa. Indeed it was shown that coupling of an NLS to plasmids enhanced the nuclear accumulation and expression of plasmid DNA. Preferably therefore, an NLS is coupled to any expression construct for use in the conjugates of the invention.

A large variety of methods for conjugation of ligands with the agents or carriers are known in the art. Such methods are e.g. described by Hermanson (1996, Bioconjugate Techniques, Academic Press), in U.S. 6,180,084 and U.S. 6,264,914 and include e.g. methods used to link haptens to carrier proteins as routinely used in applied immunology (see Harlow and Lane, 1988, "Antibodies: A laboratory manual", Cold Spring Harbor Laboratory Press, Cold Spring Harbor, NY). It is recognised that, in some cases, a ligand or agent may lose efficacy or functionality upon conjugation depending, e.g., on the conjugation procedure or the chemical group utilised therein. However, given the large variety of methods for conjugation the skilled person is able to find a conjugation method that does not or least affects the efficacy or functionality of the entities to be conjugated. Suitable methods for conjugation of a ligand with an agent or carrier include e.g. carbodiimide conjugation (Bauminger and Wilchek, 1980, Meth. Enzymol. 70: 151-159). Alternatively, an agent or carrier can be coupled to a ligand as described by Nagy et al., Proc. Natl. Acad. Sci. USA 93:7269-7273 (1996); and Nagy et al., Proc. Natl. Acad. Sci. USA 95:1794-1799 (1998). Other methods for conjugating that may suitable be used are e.g. sodium periodate oxidation followed by reductive alkylation of appropriate reactants and glutaraldehyde crosslinking. A particularly advantageous method of conjugation may be applied when both the ligand as well as the agent or carrier are (poly)peptides. In such instances the two entities may be synthesised as a single (poly)peptide chain comprising the amino acid sequences of both the ligand and the peptide agent or carrier. In addition to covalent bonding, in a conjugate according to the invention the agent or carrier may also be directly conjugated to the ligand molecule by non-specific or specific protein-protein interaction, non-covalent bonding and/or coordinating chemical bonding, which conjugation may optionally be effected via a spacer or linker that is bound to the agent and the ligand.

In another aspect, the invention discloses a conjugate of the invention as defined above, for use in the treatment and/or prevention of a viral infection. A conjugate of the invention is used in the manufacture of a medicament for the treatment and/or prevention of a viral infection. Similarly methods for the treatment and/or prevention of a viral infection, wherein an effective dose of a conjugate of the invention is administered to a subject in need thereof are disclosed. The subject in need of treatment or prevention of a viral infection may be a vertebrate, mammal, or, preferably a human.

### Viral infections and associated conditions

The following paragraphs provides a description of the various viruses, viral diseases and associated conditions that, in various embodiments, may be treated and/or prevented with the conjugates of the invention comprising an intracellularly active antiviral agent. Many viruses encode for their own RNA/DNA polymerases or other proteins or enzymes necessary for their replication or function, such as proteases, mRNA capping enzymes, neuramidases, ribonucleases, and kinases and are as such amendable for treatment with intracellularly active antiviral agents. Moreover, the over 100 viruses that are capable of causing acute viral encephalitis especially require treatments with intracellularly active antiviral agents that can reach cell populations in the CNS, into and across the blood-brain barrier. Well known examples of such viruses are arboviruses (flaviviridae, bunyaviridae, togaviridae), enteroviruses, mumps, influenza, rabies and herpes viruses like varicella, herpes simplex virus, cytomegalovirus. Therefore, in a preferred embodiment, the conjugates are used in methods for treatment and/or prevention of a viral infection of cells of the CNS. The viral infection of the CNS may e.g. be at least one of a viral meningitis, encephalitis, encephalomyelitis, and progressive multifocal leukoencephalopathy.

In a preferred embodiment, the viral infection is caused by an arbovirus selected from one of the following families: *Flαviviridαe, Bunyαviridαe* and *Togαviridαe.* Flaviviruses are enveloped, positive single-stranded RNA viruses that belong, together with the Hepaci- and Pestiviruses (hepatitis C virus (HCV), hepatitis B virus (HBV), bovine viral diarrhea virus (BVDV), classical swine fever virus (CSFV), border disease virus (BDV), and hepatitis G virus/GB-virus C (HGV/GBV-C), to the family of the Flaviviridae. The genus Flavivirus contains (i) viruses that are transmitted by mosquitoes or ticks (arthropod-borne) and (ii) viruses with no known vector (NKV), like Modoc virus and Montana Myotis leukoencephalitis virus.

One of the most important flaviviruses causing disease in man is dengue virus (DENV), causing several hundred thousand cases of dengue hemorrhagic fever (DHF) or dengue shock syndrome (DSS), the latter with an overall case fatality rate of about 5%.

Yellow fever virus (YFV) is, despite the availability of a highly efficacious vaccine, still a leading cause of viral hemorrhagic fever (VHF) worldwide. The World Health Organization has estimated that there are annually 200,000 cases of YF, including 30,000 deaths, of which over 90% occur in Africa.

Japanese encephalitis (JE), a mosquito-borne arboviral infection, is the leading cause of viral encephalitis in Asia. According to the World Health Organization approximately 50,000 sporadic and epidemic cases of JE have been reported annually. The infection results in high mortality (30%), and about half of the survivors develop long-lasting neurological sequelae.

Murray Valley encephalitis virus (MVEV), and West Nile, St. Louis encephalitis, Alfuy, Cacipacore, Koutango, Kunjin, Rocio, Stratford, Usutu and Yaounde viruses, all belong to the JE antigenic complex and cause encephalitis in man. Although the last large epidemic caused by MVEV occurred in 1974, new cases of MVEV infection are reported regularly, especially in Western Australia. In 1996 an outbreak of West Nile (WN) encephalitis with 373 cases and 17 deaths was reported in Romania. In 1999, the disease appeared for the first time in the northeastern United States and has continued to spread across the United States and Canada. In 2003, 9388 human cases of WN fever, WN (meningo)encephalitis and 246 deaths were reported in the USA. At the beginning of 2007, the total number of deaths already counted 934. Outbreaks of WN encephalitis occurred in recent years also in Southern Russia and Israel. St. Louis encephalitis virus (SLEV) is endemic in the western United States and is responsible for severe neurological disease.

Other important flaviviruses that cause encephalitis are also responsible for high mortality rates or neurological sequelae, including tick-borne encephalitis virus (TBEV) which is believed to cause annually at least 11,000 human cases of encephalitis in Russia and about 3000 cases in the rest of Europe. Related viruses within the same group are Louping ill virus (LIV), Langat virus (LGTV), Russian spring-summer encephalitis virus (RSSEV) and Powassan virus (POWV). LIV is primarily known as a disease of sheep, but has also been shown to infect, and cause disease in deer, cattle, goats, red grouse and occasionally in man. LGTV and POWV also cause human encephalitis but, as for LIV, rarely on an epidemic scale. Three other viruses within the same group, Omsk hemorrhagic fever virus (OHFV), Kyasanur Forest disease virus (KFDV) and Alkhurma virus (ALKV), are closely related to the TBE complex viruses and cause fatal hemorrhagic fevers rather than encephalitis.

Other flaviviridae include Gadgets Gully virus (GGYV), Kadam virus (KADV), Royal Farm virus (RFV), Meaban virus (MEAV), Saumarez Reef virus (SREV), Tyuleniy virus (TYUV), Aroa virus (AROAV), Kedougou virus (KEDV), Cacipacore virus (CPCV), Koutango virus (KOUV), Usutu virus (USUV), Yaounde virus (YAOV), Kokobera virus (KOKV), Bagaza virus (BAGV), Ilheus virus (ILHV), Israel turkey meningoencephalomyelitis virus (ITV), Ntaya virus (NTAV), Tembusu virus (TMUV), Zika virus (ZIKV), Banzi virus (BANV), Bouboui virus (BOUV), Edge Hill virus (EHV), Jugra virus (JUGV), Saboya virus (SABV), Sepik virus (SEPV), Uganda S virus (UGSV), Wesselsbron virus (WESSV), Entebbe bat virus (ENTV), Yokose virus (YOKV), Apoi virus (APOIV), Cowbone Ridge virus (CRV), Jutiapa virus (JUTV), Sal Vieja virus (SVV), San Perlita virus (SPV), Bukalasa bat virus (BBV), Carey Island virus (CIV), Dakar bat virus (DBV), Phnom Penh bat virus (PPBV) and Rio Bravo virus (RBV).

Bunyaviridae family include Rift Valley fever, Crimean-Congo hemorrhagic fever (CCHF), La Crosse (LAC), hanta (causing Korean hemorrhagic fever), Punta Toro, Jamestown Canyon (JTC), California encephalitis, Trivittatus, Keystone, snowshoe hare, Slough, Melao, Oropouche, Potosi, San Angelo and sandfly fever viruses. CCHF is a zoonosis transmitted by ticks that results in severe outbreaks in humans but which is not pathogenic for ruminants, their amplificator host. Although CCHF virus is not pathogenic in animals, the disease is known as one of the most important VHFs because of its high case fatality ratio (10-40%) and its potential for nosocomial transmission. CCHF is endemic throughout Africa, the Balkans, the Middle East and Asia south of 50° latitude north, the limit of its tick reservoir, the genus Hyalomma. Reports of sporadic human cases and limited outbreaks are increasing every year. Recently outbreaks of CCHF in Afghanistan (2001-2006), Iran (2001), Kazakhstan (2005), Kosovo (2001), Mauritania (2002-2003), Pakistan (2001-2006), Russia (2006), Senegal (2004 with one human case imported to France), South Africa (2006), Sudan (2004), Tajikistan (2002-2004), and Turkey (2003-2006) have drawn international attention to this emerging problem. In these endemic areas, ecological changes, poverty and social instability, insufficient medical equipment together with absence of infection control standard precautions have all contributed to the increased transmission of the CCHF virus in its natural environment, in the community or in hospital settings. Absence of available and affordable therapy still limits outbreak control activities. There is currently no specific antiviral therapy for CCHF. However, ribavirin has been shown to inhibit in-vitro viral replication in Vero cells and reduced the mean time to death in a suckling mouse model of CCHF. Additionally, several case reports have been published that suggest oral or intravenous ribavirin is effective for treating CCHF infections. All published reports showed a clear benefit in patients with confirmed CCHF treated with ribavirin (intravenous and oral administration). There were no major side effects or mortality associated with ribavirin treatment. The results of all these studies are limited by their design and sample size.

Hemorrhagic fever with renal syndrome (HFRS) is a group of clinically similar illnesses caused by hantaviruses from the Bunyaviridae family of viruses. HFRS includes diseases such as Korean hemorrhagic fever, epidemic hemorrhagic fever, and nephropathis epidemica. The viruses that cause HFRS include Hantaan, Dobrava-Belgrade, Seoul, and Puumala. HFRS is found throughout the world. Hantaan virus is widely distributed in eastern Asia, particularly in China, Russia, and on the Korean peninsular. Puumala virus is found in Scandinavia, western Europe, and Russia. Dobrava virus is found primarily in the Balkans, and Seoul virus is found worldwide. Ribavirin was demonstrated to have anti-hantaviral effect both in vitro and in vivo. Ribavirin is often used in treatment of HFRS in China and clinical trials have shown that ribavirin therapy can significantly reduce HFRS-associated mortality. A prospective, randomized, double-blind, concurrent, placebo- controlled clinical trial of intravenous ribavirin in 242 patients with serologically confirmed hemorrhagic fever with renal syndrome (HFRS) was carried out in the People's Republic of China. Mortality was significantly reduced (sevenfold decrease in risk) among ribavirin-treated patients. Ribavirin therapy resulted in a significant risk reduction of entering the oliguric phase and experiencing hemorrhage. The only ribavirin-related side effect was a fully reversible anemia after completion of therapy. The effectiveness of ribavirin therapy for HFRS was also demonstrated by different Chinese investigators.

Those viruses particularly considered in the Togaviridae family include Venezuelan equine encephalomyelitis (VEE), Eastern equine encephalitis (EEE) and Western equine encephalitis (WEE) viruses.

Other viruses that would require treatments with intracellularly and/or CNS active antiviral agents are arenaviridae (Pichinde virus, Lymphocytic Choriomeningitis Virus (LCMV), Lassa virus (causing Lassa fever) and Argentine hemorrhagic fever (AHF)), paramyxoviridae (respiratory syncytial virus (RSV), measles virus (causing subacute sclerosing panencephalitis), mumps virus), herpesviridae (varicella-zoster (VZV), herpes simplex virus (HSV), human herpes virus-6 (HHV-6), cytomegalovirus (CMV), and Epstein Barr virus (EBV)), orthomyxoviridae (influenza A and B virus), picornaviridae (enteroviruses (3 polioviruses (PV), 28 echoviruses (ECV), 23 group A and 6 group B coxsackieviruses (CVA and CBV, respectively), and Theiler's virus), and 4 numbered enteroviruses), poxviridae (smallpox (variola), cowpox virus (CV), camelpox, monkeypox, and vaccinia viruses), reoviridae (bluetongue virus, rotavirus, simian (SA11) rotavirus and Colorado tick fever virus (CTFV)), polyomaviridae (JC Virus (JCV, causing PML in immune compromised patients), BK Virus (BKV) and simian virus 40 (SV40)), filoviridae (Marburg virus, Ebola virus), rhabdoviridae (rabies), retroviridae (Human T-lymphotropic virus (HTLV, type I and II), Human immunodeficiency virus (HIV, type I and II)), coronaviridae (coronavirus (causing SARS), torovirus), adenoviridae and iridoviridae.

Lassa virus hemorrhagic fever is an acute illness that occurs in West Africa. The virus is a single-stranded RNA virus belonging to the virus family Arenaviridae. Lassa fever is known to be endemic in Guinea (Conakry), Liberia, Sierra Leone and parts of Nigeria, but probably exists in other West African countries as well. Some studies indicate that 300 000 to 500 000 cases of Lassa fever and 5000 deaths occur yearly across West Africa. The overall case-fatality rate is 1%-2% in the community, up to 15%-25% among hospitalized patients, and up to 50%-60% during outbreaks. Deafness has been documented in more than 25%-30% of the patients that have recovered. Death usually occurs within 14 days of onset in fatal cases. The disease is especially severe late in pregnancy, with maternal death or fetal loss occurring in over 80% of cases during the third trimester. In a prospective study of Lassa fever in Sierra Leone, the efficacy of ribavirin (intravenous and oral administration) was evaluated and Lassa virus-convalescent plasma for the treatment of Lassa fever. It was concluded that ribavirin was effective in the treatment of Lassa fever and should be used at any point in the illness, though preferably during the first six days after onset.

Argentine hemorrhagic fever (AHF) is transmitted by rodents and caused by Junin virus, a member of the Arenaviridae family. Since the disease was first recognized in 1955, annual outbreaks have been notified without interruption, with more than 24.000 cases reported in 1993. The endemo-epidemic area of the disease is located in the humid pampa, the most fertile farm land of Argentina. AHF is a serious acute viral disease characterized by a febrile syndrome with hematological, neurological, renal and cardiovascular alterations. Without treatment, case-fatality ratio is 15-30%. Since 1992, an attenuated live vaccine against AHF has been available. The vaccine has been used in high-risk adult populations with a significant reduction in the incidence of the disease. However, even with an effective vaccine, sporadic cases and outbreaks continue to occur. The early treatment with AHF convalescent plasma is extremely effective and reduces mortality to 1%. However, this treatment is only effective if given during the first 8 day-period after onset of symptoms. In addition plasma therapy entails risk of transfusion-borne diseases and the presentation of a late neurologic syndrome (LNS) that has been occurred in 10% of treated survivors. There are only few animal and human studies on the clinical effectiveness of ribavirin in the treatment of New World Arenaviridae. These limited clinical data indicate a clear benefit of ribavirin treatment, with good tolerability and safety of the drug.

Given the broad spectrum antiviral activity of the currently marketed drugs ribavirin (with established activity against e.g., flaviviridae, rhabdoviridae, togaviridae, bunyaviridae, arenaviridae, filoviridae, poxviridae, reoviridae, picornaviridae and orthomyxoviridae) and cidofovir (with established activity against e.g., herpesviridae, polyomaviridae and poxviridae), these are very suitable drugs for improved targeted delivery and toxicity profile, especially by improving the rate of intracellular and /or CNS availability. This is also true for the more specific, widely used antiviral drugs acyclovir and ganciclovir (established selectively against herpesviridae), and zanamivir and oseltamivir (established selectively against paramyxoviridae).

In one embodiment the viral infection causes a non-chronic conditions, such as (sub)acute virus-induced disease like meningitis, encephalitis, encephalomyelitis, progressive multifocal leukoencephalopathy (PML), retinitis, nephritis, gastroenteritis, bronchiolitis, pneumonitis, severe acute respiratory syndrome (SARS), hemorrhagic fevers and the like. In another embodiment the viral infection causes a neurological or central nervous system (CNS) disorder. Traditional treatment of non-chronic conditions, such as (sub)acute virus-induced disease like meningitis, encephalitis, encephalomyelitis, progressive multifocal leukoencephalopathy (PML), retinitis, nephritis, gastroenteritis, bronchiolitis, pneumonitis, severe acute respiratory syndrome (SARS), hemorrhagic fevers and the like, is often not in time. Further, such traditional treatments are usually limited by toxicity, with the most frequent side effects being the development of hemolytic anemia or kidney damage, requiring either dose reduction or discontinuation in certain patients, with a consequent reduction in response to therapy. In a preferred, the disorder is a subacute or an acute disease. In a more preferred embodiment the disorder is viral encephalitis.

Given the distinct mechanism of action, intracellularly active antiviral agents can be effectively co-administered/treated with other classes of antiviral medications like type I interferons, or inducers thereof viral entry and fusion inhibitors, vaccination programs, or with anti-inflammatory therapies like with glucocorticoids and the like,

CRM197-RBV conjugate, CRM197-PEG-liposome comprising RBV or CRM197-PEG-PEI anti-JEV DNAzymes polyplex may be co-administered with dexamethasone or interferon alpha-2a or both.

### Targeting to and/or across blood-tissue barriers

In another aspect, a method of targeted drug delivery of an effective amount of an antiviral agent, or a pharmaceutical acceptable carrier comprising an antiviral agent, to a target site that is protected by a specific blood-tissue barriers like e.g., the CNS, the blood-brain barrier (BBB), the retina and the testes, is provided wherein: a) the antiviral agent or the pharmaceutical acceptable carrier is conjugated to a ligand, that facilitates the specific binding to and internalization by an internalizing uptake receptor of the target site, thereby forming the conjugate as defined above; and b) the antiviral agent is delivered at the target site within a time period of about day 1 to about day 5 after administration to a person in need. In a preferred embodiment, the blood-tissue barrier, e.g. blood-brain barrier in the method is not disrupted by administration of agents disrupting the blood-tissue barrier. In another preferred embodiment, the time-period is of about day 1 to about day 7, more preferably of about day 1 to about day 10, even more preferably of about day 1 to about day 14, most preferably of about day 1 to about day 21.

The following paragraphs relate to various embodiments of the invention concerning the active targeting to target sites protected by specific blood-tissue barriers like e.g., the CNS, the blood-brain barrier (BBB), the retina and the testes, by receptor-mediated transcytosis. In a preferred embodiment of the invention, the receptor that mediates at least one of endocytosis and transcytosis is located in (the luminal side of) capillaries in the brain. In general, without wishing to be bound to any theory, receptor-mediated transcytosis occurs in three steps: receptor-mediated endocytosis of the agent at the luminal (blood) side, movement through the endothelial cytoplasm, and exocytosis of the drug at the abluminal (brain) side of the brain capillary endothelium. Upon receptor-ligand internalization, chlathrin-coated vesicles are formed, which are approximately 120 nm in diameter. These vesicles may transport their content to the other side of the cell or go into a route leading to protein degradation. Indeed, at least two important routes for degrading proteins have been identified, including the lysosomal and the ubiquitin-proteasome route. Therefore, to escape from the endosomallysosomal system, mechanisms have been applied to ensure release of the drug into the cytosol. These include the application of pH-sensitive liposomes or cationic molecules. The diphtheria toxin that is also applicable as a targeting ligand, and is discussed later, has an intrinsic lysosomal escape mechanism. Nevertheless, with or without application of lysosomal escape mechanisms, protein delivery to the brain has been shown to be effective. Therefore, receptor-mediated transcytosis allows the specific targeting of larger drug molecules or drug-carrying particles (such as liposomes, polymer systems, nanoparticles) to the brain. In a preferred embodiment, at least one of the receptor that mediates at least one of endocytosis and transcytosis, the ligand and the pharmaceutical acceptable carrier is selected to bypass lysosomal degradation of the antiviral agent in the cell.

### Gene therapy

Some aspects of the invention concern the use of expression vectors comprising the nucleotide sequences encoding an antiviral agent comprising an oligo- or poly-nucleotide as defined above, wherein the vector is a vector that is suitable for gene therapy. Vectors that are suitable for gene therapy are described in Anderson 1998, Nature 392: 25-30; Walther and Stein, 2000, Drugs 60: 249-71; Kay et al., 2001, Nat. Med. 7: 33-40; Russell, 2000, J. Gen. Virol. 81: 2573-604; Amado and Chen, 1999, Science 285: 674-6; Federico, 1999, Curr. Opin. Biotechnol.10: 448-53; Vigna and Naldini, 2000, J. Gene Med. 2: 308-16; Marin et al., 1997, Mol. Med. Today 3: 396-403; Peng and Russell, 1999, Curr. Opin. Biotechnol. 10: 454-7; Sommerfelt, 1999, J. Gen. Virol. 80: 3049-64; Reiser, 2000, Gene Ther. 7: 910-3; and references cited therein. Particularly suitable gene therapy vectors include Adenoviral and Adeno-associated virus (AAV) vectors. These vectors infect a wide number of dividing and non-dividing cell types. In addition adenoviral vectors are capable of high levels of transgene expression. However, because of the episomal nature of the adenoviral and AAV vectors after cell entry, these viral vectors are most suited for therapeutic applications requiring only transient expression of the transgene (Russell, 2000, J. Gen. Virol. 81: 2573-2604) as indicated above. Preferred adenoviral vectors are modified to reduce the host response as reviewed by Russell (2000, supra).

Generally, gene therapy vectors will be as the expression vectors described above in the sense that they comprise the nucleotide sequence encoding antiviral agent to be expressed, whereby the nucleotide sequence is operably linked to the appropriate regulatory sequences as indicated above. Such regulatory sequence will at least comprise a promoter sequence. As used herein, the term "promoter" refers to a nucleic acid fragment that functions to control the transcription of one or more genes, located upstream with respect to the direction of transcription of the transcription initiation site of the gene, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active under most physiological and developmental conditions. An "inducible" promoter is a promoter that is regulated depending on physiological or developmental conditions. A "tissue specific" promoter is only active in specific types of differentiated cells/tissues. Suitable promoters for expression of the nucleotide sequence encoding the polypeptide from gene therapy vectors include e.g. cytomegalovirus (CMV) intermediate early promoter, viral long terminal repeat promoters (LTRs), such as those from murine moloney leukaemia virus (MMLV) rous sarcoma virus, or HTLV-1 , the simian virus 40 (SV 40) early promoter and the herpes simplex virus thymidine kinase promoter.

Several inducible promoter systems have been described that may be induced by the administration of small organic or inorganic compounds. Such inducible promoters include those controlled by heavy metals, such as the metallothionine promoter (Brinster et al. 1982 Nature 296: 39-42; Mayo et al. 1982 Cell 29: 99-108), RU-486 (a progesterone antagonist) (Wang et al. 1994 Proc. Natl. Acad. Sci. USA 91: 8180-8184), steroids (Mader and White, 1993 Proc. Natl. Acad. Sci. USA 90: 5603-5607), tetracycline (Gossen and Bujard 1992 Proc. Natl. Acad. Sci. USA 89: 5547-5551; U.S. Pat. No. 5,464,758; Furth et al. 1994 Proc. Natl. Acad. Sci. USA 91: 9302-9306; Howe et al. 1995 J. Biol. Chem. 270: 14168-14174; Resnitzky et al. 1994 Mol. Cell. Biol. 14: 1669-1679; Shockett et al. 1995 Proc. Natl. Acad. Sci. USA 92: 6522-6526) and the tTAER system that is based on the multi-chimeric transactivator composed of a tetR polypeptide, as activation domain of VP 16, and a ligand binding domain of an estrogen receptor (Yee et al., 2002, US 6,432,705).

The gene therapy vector may optionally comprise a second or one or more further nucleotide sequence coding for a second or further protein. The second or further protein may be a (selectable) marker protein that allows for the identification, selection and/or screening for cells containing the expression construct. Suitable marker proteins for this purpose are e.g. fluorescent proteins such as e.g. the green GFP, and the selectable marker genes HSV thymidine kinase (for selection on HAT medium), bacterial hygromycin B phosphotransferase (for selection on hygromycin B), Tn5 aminoglycoside phosphotransferase (for selection on G418), and dihydrofolate reductase (DHFR) (for selection on methotrexate), CD20, the low affinity nerve growth factor gene. Sources for obtaining these marker genes and methods for their use are provided in Sambrook and Russel (2001) "Molecular Cloning: A Laboratory Manual (3rd edition), Cold Spring Harbor Laboratory, Cold Spring Harbor Laboratory Press, New York.

Alternatively, the second or further nucleotide sequence may encode a protein that provides for fail-safe mechanism that allows to cure a subject from the transgenic cells, if deemed necessary. Such a nucleotide sequence, often referred to as a suicide gene, encodes a protein that is capable of converting a prodrug into a toxic substance that is capable of killing the transgenic cells in which the protein is expressed. Suitable examples of such suicide genes include e.g. the E.coli cytosine deaminase gene or one of the thymidine kinase genes from Herpes Simplex Virus, Cytomegalovirus and Varicella-Zoster virus, in which case ganciclovir may be used as prodrug to kill the IL-10 transgenic cells in the subject (see e.g. Clair et al., 1987, Antimicrob. Agents Chemother. 31: 844-849). The gene therapy vectors are preferably formulated in a pharmaceutical composition comprising a suitable pharmaceutical carrier as defined below.

### Antibodies

Antibodies or antibody-fragments may be a component part of the conjugates of the invention. Preferably the antibody or fragment thereof is a monoclonal antibody (MAb). MAbs to complement components can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, (Cold Spring Harbor Laboratory Press, 2nd ed. 1988); Hammerling, et al., in: Monoclonal Antibodies and T-Cell Hybridomas 563-681 (Elsevier, N.Y., 1981). For treating humans, the anti-complement MAbs would preferably be used as chimeric, deimmunised, humanised or human antibodies. Such antibodies can reduce immunogenicity and thus avoid human anti-mouse antibody (HAMA) response. It is preferable that the antibody be IgG4, IgG2, or other genetically mutated IgG or IgM which does not augment antibody-dependent cellular cytotoxicity (S.M. Canfield and S.L. Morrison, J. Exp. Med., 1991: 173: 1483-1491) and complement mediated cytolysis (Y.Xu et al., J. Biol. Chem., 1994: 269: 3468-3474; V.L. Pulito et al., J. Immunol., 1996; 156: 2840-2850). Chimeric antibodies are produced by recombinant processes well known in the art, and have an animal variable region and a human constant region. Humanised antibodies have a greater degree of human peptide sequences than do chimeric antibodies. In a humanised antibody, only the complementarity determining regions (CDRs) which are responsible for antigen binding and specificity are animal derived and have an amino acid sequence corresponding to the animal antibody, and substantially all of the remaining portions of the molecule (except, in some cases, small portions of the framework regions within the variable region) are human derived and correspond in amino acid sequence to a human antibody. See L. Riechmann et al., Nature, 1988; 332: 323-327; G. Winter, United States Patent No. 5,225,539; C.Queen et al., U.S. 5,530,101. Deimmunised antibodies are antibodies in which the T and B cell epitopes have been eliminated, as described in WO9852976. They have reduced immunogenicity when applied in vivo. Human antibodies can be made by several different ways, including by use of human immunoglobulin expression libraries (Stratagene Corp., La Jolla, California) to produce fragments of human antibodies (VH, VL, Fv, Fd, Fab, or (Fab')2, and using these fragments to construct whole human antibodies using techniques similar to those for producing chimeric antibodies. Human antibodies can also be produced in transgenic mice with a human immunoglobulin genome. Such mice are available from Abgenix, Inc., Fremont, California, and Medarex, Inc., Annandale, New Jersey. One can also create single peptide chain binding molecules in which the heavy and light chain Fv regions are connected. Single chain antibodies ("ScFv") and the method of their construction are described in U.S. Patent No. 4,946,778. Alternatively, Fab can be constructed and expressed by similar means (M.J. Evans et al., J. Immunol. Meth., 1995; 184: 123-138). Another class of antibodies that may be used in the context of the present invention are heavy chain antibodies and derivatives thereof. Such single-chain heavy chain antibodies naturally occur in e.g. Camelidae and their isolated variable domains are generally referred to as "VHH domains" or "nanobodies". Methods for obtaining heavy chain antibodies and the variable domains are inter alia provided in the following references: WO 94/04678, WO 95/04079, WO 96/34103, WO 94/25591, WO 99/37681, WO 00/40968, WO 00/43507, WO 00/65057, WO 01/40310, WO 01/44301, EP 1134231, WO 02/48193, WO 97/49805, WO 01/21817, WO 03/035694, WO 03/054016, WO 03/055527, WO 03/050531, WO 01/90190, WO 03/025020, WO 04/041867, WO 04/041862, WO04/041865, WO 04/041863, WO 04/062551. All of the wholly and partially human antibodies are less immunogenic than wholly murine MAbs (or MAbs from other non-human animals), and the fragments and single chain antibodies are also less immunogenic. All these types of antibodies are therefore less likely to evoke an immune or allergic response. Consequently, they are better suited for in vivo administration in humans than wholly animal antibodies, especially when repeated or long-term administration is necessary. In addition, the smaller size of the antibody fragment may help improve tissue bioavailability, which may be critical for better dose accumulation in acute disease indications, such as tumour treatment or some viral infections.

### Pharmaceutical compositions

Further disclosed is a pharmaceutical preparation comprising as active ingredient a conjugate as herein defined above. The composition preferably at least comprises a pharmaceutically acceptable carrier (other than the carrier in the conjugate) in addition to the active ingredient (the conjugate). In some methods, the conjugate comprises a polypeptide or antibody of the invention as purified from mammalian, insect or microbial cell cultures, from milk of transgenic mammals or other source is administered in purified form together with a pharmaceutical carrier as a pharmaceutical composition. Methods of producing pharmaceutical compositions comprising polypeptides are described in US Patents No.'s 5,789,543 and 6,207,718. The preferred form depends on the intended mode of administration and therapeutic application. The pharmaceutical carrier can be any compatible, non-toxic substance suitable to deliver the polypeptides, antibodies or gene therapy vectors to the patient. Sterile water, alcohol, fats, waxes, and inert solids may be used as the carrier. Pharmaceutically acceptable adjuvants, buffering agents, dispersing agents, and the like, may also be incorporated into the pharmaceutical compositions. The concentration of the conjugate of the invention in the pharmaceutical composition can vary widely, i. e., from less than about 0.1 % by weight, usually being at least about 1% by weight to as much as 20% by weight or more. For oral administration, the active ingredient can be administered in solid dosage forms, such as capsules, tablets, and powders, or in liquid dosage forms, such as elixirs, syrups, and suspensions. Active component(s) can be encapsulated in gelatin capsules together with inactive ingredients and powdered carriers, such as glucose, lactose, sucrose, mannitol, starch, cellulose or cellulose derivatives, magnesium stearate, stearic acid, sodium saccharin, talcum, magnesium carbonate and the like. Examples of additional inactive ingredients that may be added to provide desirable colour, taste, stability, buffering capacity, dispersion or other known desirable features are red iron oxide, silica gel, sodium lauryl sulfate, titanium dioxide, edible white ink and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric-coated for selective disintegration in the gastrointestinal tract. Liquid dosage forms for oral administration can contain colouring and flavouring to increase patient acceptance. The conjugates of the invention are preferably administered parentally. Preparation with the conjugates for parental administration must be sterile. Sterilisation is readily accomplished by filtration through sterile filtration membranes, prior to or following lyophilisation and reconstitution. The parental route for administration of the conjugates is in accord with known methods, e.g. injection or infusion by intravenous, intraperitoneal, intramuscular, intraarterial, intralesional, intracranial, intrathecal, transdermal, nasal, buccal, rectal, or vaginal routes. The conjugate is administered continuously by infusion or by bolus injection. A typical composition for intravenous infusion could be made up to contain 10 to 50 ml of sterile 0.9% NaCl or 5% glucose optionally supplemented with a 20% albumin solution and the required dose of the conjugate. A typical pharmaceutical composition for intramuscular injection would be made up to contain, for example, 1 - 10 ml of sterile buffered water and the required dose of the conjugate of the invention. Methods for preparing parenterally administrable compositions are well known in the art and described in more detail in various sources, including, for example, Remington's Pharmaceutical Science (15th ed., Mack Publishing, Easton, PA, 1980).

For therapeutic applications, the pharmaceutical compositions are administered to a patient suffering from a viral infection or associated condition in an amount sufficient to reduce the severity of symptoms and/or prevent or arrest further development of symptoms. An amount adequate to accomplish this is defined as a "therapeutically-" or "prophylactically-effective dose". Such effective dosages will depend on the severity of the condition and on the general state of the patient's health.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

### Description of the figures

Figure 1 shows a representative picture of the receptor-specific uptake and cellular (peri)endosomal localization of CRM197-FITC in LLC-PK1 cells.
Figure 2 shows a representative picture of the receptor-specific uptake and cellular (peri)endosomal localization of RBV-loaded CRM197-PEG-liposomes (labelled with Rho-PE) in LLC-PK1 cells.
Figure 3 shows a representative picture of the uptake of glutathione-PEG-liposomes (labelled with Rho-PE) in BCEC.
Figure 4 shows representative pictures of the receptor-specific targeting to the indicated hamster tissues and organs of CRM197-FITC, and compared to HRP-FITC, 90 minutes after a single intravenous bolus injection.
Figure 5 shows representative pictures of the receptor-specific targeting to the indicated hamster tissues and organs of CRM197-PEG-liposomes, and compared to control PEG-liposomes, 24 hours after the last intravenous daily bolus injection for 8 consecutive days.
Figure 6 shows representative pictures of the receptor-specific targeting to the indicated hamster tissues and organs of glutathione-PEG-liposomes, and compared to control PEG-liposomes, 24 hours after the last intravenous daily bolus injection for 9 consecutive days.

### Examples

### Example 1

### Conjugation of antiviral agents to receptor-specific ligands

As an example of antiviral conjugation to receptor-specific ligands, the preferred method of conjugation of ribavirin to CRM197 is disclosed.

The conjugation of ribavirin to CRM197 is modified from Brookes et al. (2006, Bioconjugate Chem., 17: 530-537), is prepared by reaction of RBV with phosphorus oxychloride (POC13) and trimethyl phosphate (TMP), with progress of the reaction monitored by C18 reverse-phase HPLC. RBV (2 mmol) is reacted with POC13 (8 mmol) and purified water (2 mmol) in 8.3 mL of TMP. Following completion of the reaction (5 h), the product is poured over 20 g of ice and 2 N sodium hydroxide solution is added to bring the pH up to 3. The product is allowed to hydrolyze overnight at room temperature. The hydrolyzed product is extracted with 2 × 20 mL portions of chloroform. The product RBV-P in chloroform is mixed with 10 g of fine charcoal (100-400 mesh). The reaction mixture/charcoal slurry is centrifuged at 2000g for 15 min, and the supernatant is recovered. The wash steps are repeated until no inorganic phosphate (Pi) can be detected in the supernatant by C18 reversed-phase HPLC or by the Ames method. The charcoal is extracted three times with ethanol/water/ammonium hydroxide (10:10:1), and the pooled extract is evaporated to dryness. The resulting RBV-P ammonium salt is converted to the free acid by ion exchange using BioRAD AG 50W-X2 (H form) resin and elution of product with water according to the method by Streeter. The isolated yield after purification is 70%. The RBV-P is characterized using two assays: C18 reverse-phase HPLC quantification of the RBV released by enzymatic cleavage using acid phosphatase (acid phosphatase assay), and quantification of total inorganic phosphate (Pi) by the Ames method. Purified RBV-P is converted to ribavirin-5'-monophosphorimidazolide (RBV-P-Im) according to the procedure of Fiume with slight modifications. The reaction is performed under dry nitrogen using anhydrous solvents. RBV-P (324 mg, 1 mmol) is dissolved in 10 mL of N,N'-dimethylformamide (DMF). Carbonyldiimidazole (CDI, 5 mmol) dissolved in 5 mL of DMF is added to the RBV-P solution with stirring, followed by addition of 5 mmol of imidazole freshly predissolved in 5 mL of DMF. The reaction mixture is stirred at room temperature for 45 min followed by removal of the DMF by evaporation. The resulting waxy solid is dissolved in 2 mL of ethanol, followed by precipitation of the RBV-P-Im product by slow addition of 20 mL of ether. The precipitate is washed twice with ether, and residual ether is evaporated using a gentle stream of dry nitrogen. The RBV-P-Im is isolated in 90% yield and used immediately for conjugation to CRM197. CRM197 (667 nmol, 40 mg) (10 mL of a 4 mg/mL solution in purified water) is mixed with 10 µmol (3.74 mg) of RBV-P-Im dissolved in 860 µL of 0.1 M sodium bicarbonate, pH 9.5, buffer (150:1 ratio of RBV-P-Im to CRM197). The pH of the reaction mixture is maintained at pH 9.5-9.6 over the first hour by addition of 0.2 M sodium carbonate solution as required. The degree of CRM197 modification is monitored by anion exchange HPLC. The final reaction mixture is purified by dialysis (10 kDa molecular weight cutoff) against PBS (3 × 0.5 L exchanges), sterile filtered (0.2 µm filter), and stored at 4°C.

Similar conjugation chemistry is applied to the other herein disclosed nucleoside analogues and similar antiviral agents, and the other herein disclosed receptor-specific ligands for intracellular targeting.

In order to visualize the receptor-specific cellular uptake, as well as the in vivo pharmacokinetics and biodistribution of CRM197 conjugated to a hydrophilic antiviral agent (like most nucleoside analogues, including ribavirin and the like), CRM197 was labelled with the hydrophilic fluorescent dye fluorescein isothiocyanate (FITC). For this, CRM197 (100 nmol, 6 mg) was dissolved in 1.2 mL PBS and 120 µL 1M NaHCO3 pH 9.0. FITC (2 µmol, 78 µl of a freshly prepared stock of 10 mg/ mL DMSO) was added and the solution was stirred, in the dark, for 1 hr at room temperature. The excess of FITC was removed by ultracentrifugation (Zebra™, Pierce, Rockford, USA) after which the solution was stored in the dark at 4°C. The number of FITC molecules per molecule of CRM197 (3 to 6) was determined by measuring the solution at 494 nm. The same labeling procedure was applied to horseradish peroxidase (HRP) as a control protein.

### Example 2

### Conjugation of receptor-specific ligands to nanocontainers containing antiviral agents

As an example of antiviral agent containing nanocontainers coated with receptor-specific ligands, the preferred method of conjugation of CRM197 to RBV-loaded PEGylated liposomes is disclosed.

Liposomes consisted of 1,2-dipalmitoyl-sn-glycero-3-phosphocholine (DPPC) and cholesterol (Chol) in a molar ration of 2.0:1.5. Components were dissolved in CHCl3 MeOH (1:1 v:v). A lipid film was prepared of DPPC (50 µmol) and Chol (37.5 µmol) by evaporation of the solvents under reduced pressure. When necessary dicetyl phosphate (DP) (molar ratio 0.22) was added to the mixture. The lipids were hydrated in 1 mL 100 to 120 mg/mL RBV (or up to >500 mg/ml by heating the solution to 50°C) in PBS containing 3.5 mol% DSPE-PEG-MAL (Mw 3400) and 3.5 mol% DSPE-mPEG (Mw 2000). After vortexing the vesicles were extruded through two polycarbonate membranes of 200 nm pore diameter (9x), 100 nm (9x) and finally 50 nm (9x) at a temperature of 42°C. Liposomes were used directly for conjugation to CRM197. Hereto, CRM197 was modified with Traut's reagent (2-iminothiolane.HCl =2-IT, 15 equivalents) for 1 hr at room temperature in 160 mM borate buffer pH 8.0 containing 1 mM EDTA. The excess of 2-IT was removed by ultracentrifugation (Zebra™ column, Pierce, Rockford, USA). Per µmol phospholipids 50-100 µg modified CRM197 was added for overnight conjugation at 4°C while mixing. Alternatively, DSPE-PEG-CRM197 was synthesized before preparation of the liposomes using DSPE-PEG-MAL and 2-IT modified CRM197. DSPE-PEG-CRM197 was added either to the hydrated lipid mixture before extrusion or after extrusion by incubation at 25°C up to 55°C for 2 up to 24 hours (depending on the temperature sensitivity of the payload), in order to obtain the optimal incorporation grade of the targeting moiety to the liposome. Unbound CRM197 and free RBV were removed using Sephrose CL 4B column or via ultracentrifugation. Liposomes were characterized by measuring particle size (100-119 nm p.i. 0.07-0.19 on a Malvern Zetasizer 300 HAS), zeta potential (-18 / -9mV ± 6.5 on a Malvern Zetasizer 300 HAS), phospholipid content (14-21 mM using the Phopholipids B kit of Wako Chemicals GmbH) and protein content (0.2-0.6 mol% CRM197 based on a Modified Lowry kit of Pierce), and drug (RBV) loading (5-21%, determined at 206-210 nm in a iso-propylalcohol solution an a Agilent 8453 UV/VIS spectrometer).

Alternatively, DSPE-PEG-CRM197 was replaced by DSPE-PEG-glutathione, which was synthesized before preparation of the liposomes using DSPE-PEG-MAL and fresh solutions of reduced glutathione (rendering a MAL-reactive thiol group in the cysteine moiety of the tri-peptide).

Similar liposomal entrapment is applied to the other herein disclosed nucleoside analogues and similar antiviral agents, and similar conjugation chemistry the other herein disclosed receptor-specific ligands for intracellular targeting. In addition, similar liposomal entrapment is applied to the nucleic acid-based antiviral drugs, with additional enrichment of nucleic acid entrapment by addition of a cationic derivative of cholesterol (DC-Chol) to the liposomes, as detailed in Gao and Huang, 1991, Biochem Biophys Res Commun. 179(1):280-5, or by using amphoteric liposomes, as detailed in W02002/066012.

In order to visualize the receptor-specific cellular uptake, as well as the in vivo pharmacokinetics and biodistribution of CRM197 or glutathione conjugated to the liposome filled with an antiviral agent (like nucleoside analogues, including RBV and the like), 1,2-dioleoyl-sn-glycero-3-phosphoethanolamine-N-lissamine Rhodamine B sulfonyl (Rho-PE 0.1 mol%) was added to the lipid mixture during the preparation of the liposomes. Alternatively, liposomes are labelled with a radioactive tracer molecule.

### Example 3

### Conjugation of receptor-specific ligands to carrier for nucleid acid-based antiviral drugs

As an example of a non-viral delivery system for nucleic acid-based antiviral drugs by means of a receptor-mediated uptake mechanism, the preferred method of conjugation ofPEGylated CRM197 to polyethylenimine (PEI) is disclosed.

PEGylated complexes were prepared as follows. PEI (25 kDa, branched, 3.3 mg, 133 nmol) was dissolved in PBS at a concentration of 5 mg/mL. Poly(ethyleneglycol)-α-maleimide-ω-NHS (NHS-PEG-VS, Mw 5000, 266 nmol, 1.4 mg) was added to this solution and incubated for 1 hr at room temperature while mixing. The excess of NHS-PEG-VS was removed by ultracentrifugation (Zebra™ column, Pierce, Rockford, USA). PEI-PEG-VS was used directly for conjugation to CRM197. Hereto, CRM197 (133 nmol, 8 mg in 1.6 mL 160 mM borate buffer pH 8.0 containing 1 mM EDTA.) was modified with 2-IT (2.66 µmol, 183 µl 14.5 mM solution in 160 mM borate buffer pH 8.0) for 1 hr at room temperature. The excess of 2-IT was removed by ultracentrifugation (Zebra™ column, Pierce, Rockford, USA). Thiol activated CRM197 was conjugated overnight at 4° to PEI-PEG-VS using a 1:1 molar ratio. PBS (4 mL) was added and the solution was concentrated using a Vivaspin column (Sartorius, Epsom, UK) to remove unreacted CRM197 or PEI-PEG-VS. The purity of the conjugate was determined by SDS PAGE. Where applicable (and after complexation with the antiviral nucleic acid-based drugs), the constructs were further purified on a Sephrose CL 4B column. The same conjugation procedure was applied to HRP as a control protein.

### Example 4

### Receptor-specific cell uptake and/or transcellular transport of targeted antiviral agents

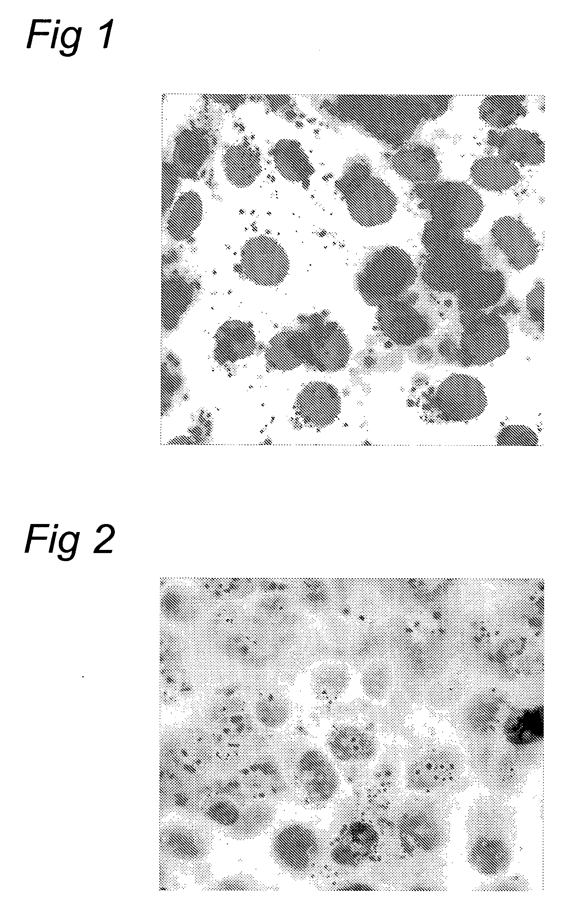

Receptor-specific cell uptake of the CRM197-RBV conjugate was visualized by analysis of the specific uptake of the CRM197-FITC conjugate, and compared to the level of uptake of HRP-FITC and free sodium fluorescein. Cells with a known expression of the DTR were used from several species and origins, including porcine kidney epithelial cells (LLC-PK1), bovine brain capillary endothelial cells (BCEC), monkey kidney fibroblast cells (COS-1), and human glioblastoma cells. In particular, LLC-PK1 cells were incubated for 1 h in 24-wells plates on 400 microliter DMEM+FCS supplemented with 100 microgram/ml heparin, before 5 microgram CRM197-FITC or HRP-FITC was added to the well. Two hours later the cells were washed 3 times and the cells were lysated in 100 microliter 0.1 N NaOH, and fluorescence was determined at 480/530 nm in a fluostar plate reader. Cell protein per well was determined with a Biorad DC assay and fluorescence in the cell lysate was calculated per mg cell protein. In a separate group of cells, an access of 100 microgram free CRM197 was added to the well, 30 minutes before the CRM197-FITC conjugate was added to the medium. The LLC-PK1 cells contained 0.54 +/- 0.02 microgram CRM197-FITC per mg cell protein, which was significantly reduced to 0.35 +/- 0.04 microgram CRM197-FITC per mg cell protein after the cells were pre-incubated with free CRM197. No HRP-FITC was found in the cell lysates. These experiments show that CRM197-FITC is specifically taken up by the DTR expressed on LLC-PK1 cells. In a separate set of experiments, LLC-PK1 cells were cultured in similar conditions on coverslips and exposed to CRM197-FITC, HRP-FITC or sodium fluorescein. After fixation and mounting with vectashield with DAPI for nuclear staining, the coverslips were analyzed on a fluorescence microscope and photographed. Figure 1 shows a representative picture of the receptor-specific uptake and cellular (peri)endosomal localization of CRM197-FITC in these cells. No fluorescence signal was found in the cells incubated with HRP-FITC and sodium fluorescein. Identical results were found on BCEC, COS-1 and human glioblastoma cells, also after several time points (up to 24 hours after exposure). Interestingly, when the incubation medium containing the CRM197-FITC was removed after 2 hours and replaced with DMEM+FCS, the fluorescent label was homogeneously distributed throughout the cytosol 4 hours later, indicating that the conjugated had escaped the endosome.

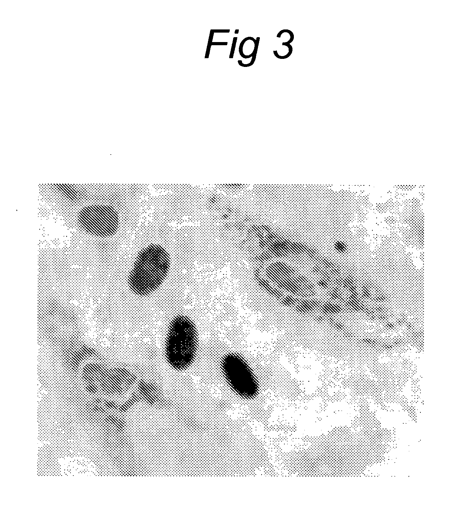

In addition, in a similar set of experiments on the BBB model described by Gaillard et al. (2001, Eur J Pharm Sci. 12(3): 215-222), BCEC exposed for up to 2 hours to RBV-loaded (between 5 and 25% of the applied concentration; about 18 mg/mL liposome solution) CRM197-PEG-liposomes (labelled with Rho-PE, between 50 and 200 nm in size, containing between 5 and 1000 CRM197 proteins), were specifically taken up by the BCEC, where the RBV-loaded PEG-liposomes could not be detected in the cells. In this BBB model, no effect on the integrity of the BBB (as determined by transendothelial electrical resistance) was observed by the RBV-loaded liposomes (containing an equivalent of 1.8 mg/mL RBV), or by free RBV (up to 1 mg/mL). In a MTT-assay on LLC-PK1 cells, however, 10 mg/mL RBV was found to be toxic after 5 h (60% cell viability). Figure 2 shows a representative picture of the receptor-specific ("dotted") uptake of the RBV-loaded CRM197-PEG-liposomes in LLC-PK1 cells after 4 hours incubation in DMEM+FCS. No receptor-specific fluorescence signal was found in the cells incubated with HRP-PEG-liposomes. In addition, similar specific uptake results were obtained with glutathione-PEG-liposomes in the BCEC, while no uptake was observed in LLC-PK1 cells, indicating that the uptake was specifically mediated by receptors expressed on the BCEC. Figure 3 shows a representative picture of the uptake of the RBV-loaded glutathione-PEG-liposomes in BCEC after 4 and 24 hours incubation in DMEM+FCS.

### Example 5

### Pharmacokinetics and biodistribution of targeted antiviral agents

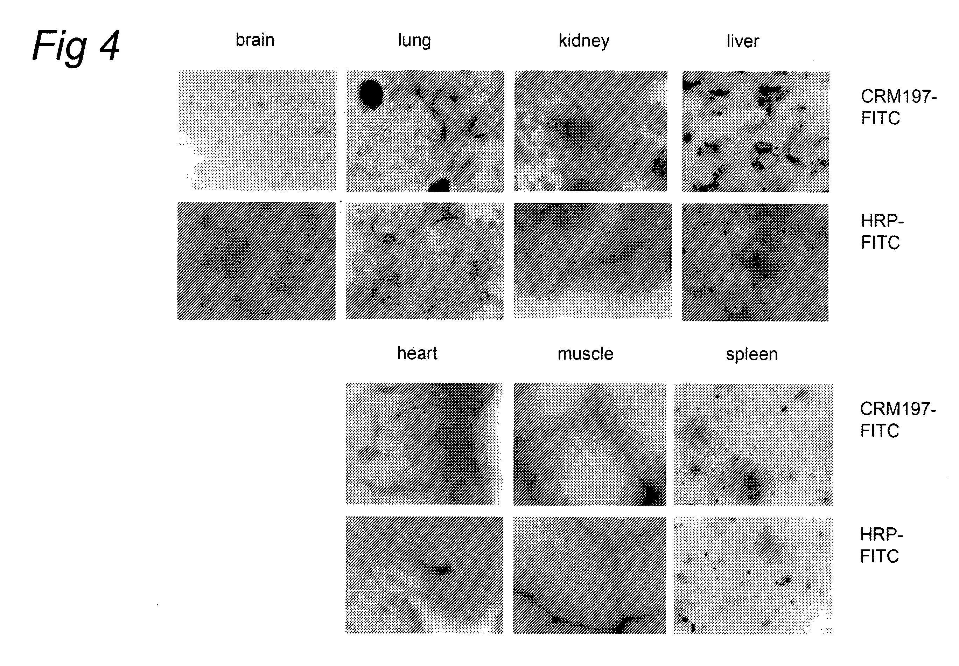

The pharmacokinetics and biodistribution of the CRM197-RBV conjugate was visualized by analysis of the CRM197-FITC conjugate after an intravenous bolus injection in hamsters, and compared to the HRP-FITC conjugate. Sixty minutes after the intravenous injection dose of 1 mg FITC-labelled protein per hamster (n=4), the plasma half-life of CRM197-FITC was calculated to be significantly higher (12 hours for CRM197-FITC, compared to 38 minutes for HRP-FITC), where at that time the AUC was essentially the same (+/- 7000 microgram*min/ml). In comparison, the half-life and AUC of a 1 mg intravenous bolus injection of sodium fluorescein in rats, was found to be 35 minutes and 13 microgram*min/ml, respectively. These pharmacokinetic properties of the CRM197 conjugate offer favorable delivery characteristics for antiviral agents. In addition, the targeted conjugates showed specific accumulation in a selection of tissues analyzed (including brain, heart, lung, liver, spleen (i.e. lymphocytes) and kidney, but not much in muscle tissue), when compared to the control (HRP) conjugates which were not (or hardly) detectable in these tissues 90 minutes after the injection (Representative pictures are shown in Figure 4).

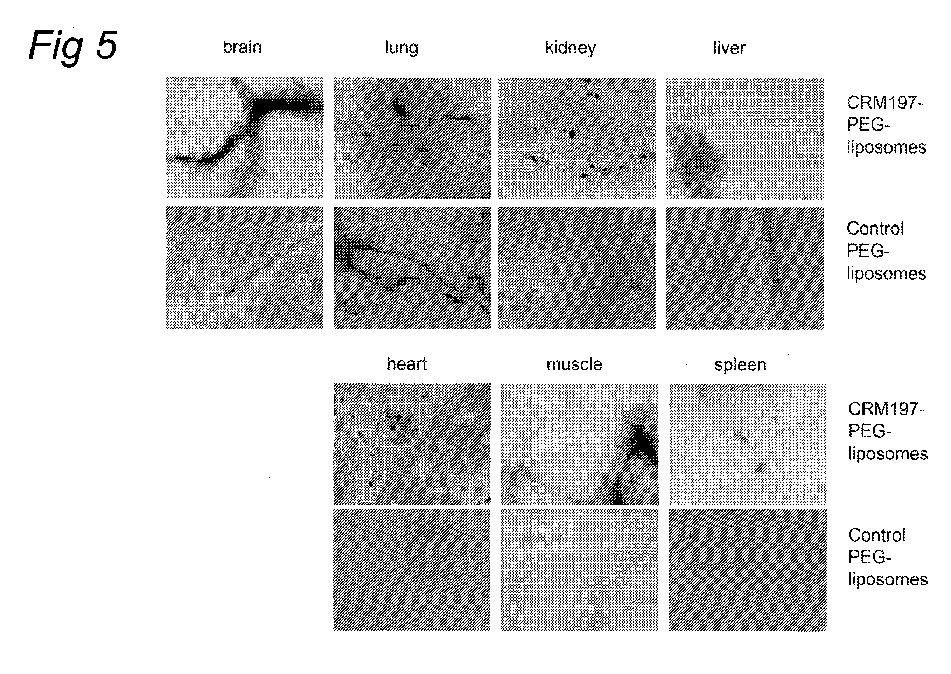

In addition, in two similar sets of experiments the biodistribution of ribavirin-loaded glutathione and CRM197-PEG-liposomes (labelled with Rho-PE) was visualized by analysis of the Rho-PE label after 8 or 9 repeated intravenous bolus injections in hamsters, and compared to control (un-targeted) PEG-liposomes. As for the CRM197-FITC conjugates, the CRM197-targeted liposomes showed specific accumulation in a selection of tissues analyzed (including brain, heart, lung, liver, spleen (i.e. lymphocytes), muscle and kidney), when compared to the control liposomes which were not (or hardly) detectable in brain, and to a far lesser extend in these tissues 24 hours after the last injection. Figure 5 shows representative pictures of these tissues.

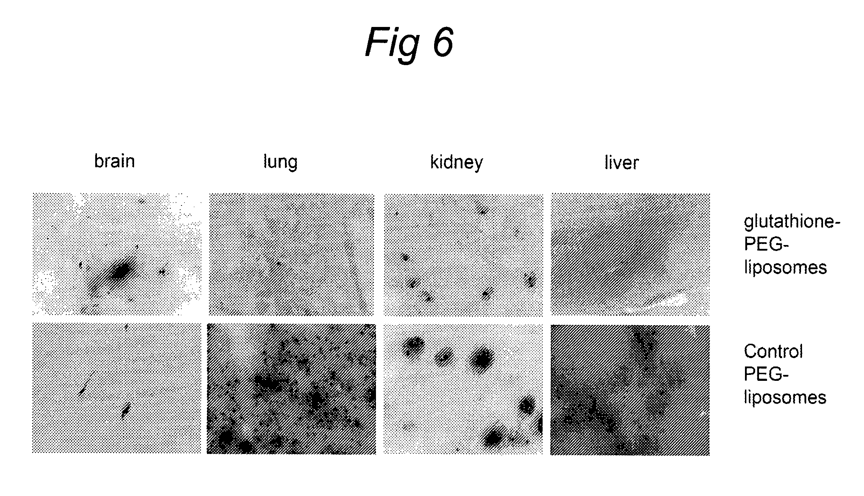

The glutathione-targeted liposomes showed a higher and specific accumulation in the perfused hamster brain, and less in a selection of other tissues analyzed (including heart, lung, liver, spleen and kidney), when compared to the control liposomes which were not (or hardly) detectable in brain, but to a relatively higher extend in lung, kidney and liver tissues 24 hours after the last injection. Figure 6 shows representative pictures of a selection of these tissues.

Given the fact that PEI itself is known to be toxic to cells and animals, the toxicity profile of CRM197-PEG-PEI LacZ plasmid polyplexes was assessed during two days after an intravenous bolus injection in hamsters, and compared to control (HRP-PEG-PEI LacZ plasmid) polyplexes. The hamsters showed no signs of distress or disease from the injections (50 microgram DNA, at a N/P ratio of 1.2), so the polyplexes were well tolerated by the animals.

### Example 6

### Antiviral activity of targeted antiviral agents in vitro

To exemplify the potency of the described invention, the receptor-specific antiviral activity is assessed in Vero cells (basically according to Leyssen et al., 2005, J Virol., 79:1943-1947). The dose-response effects of RBV, CRM197-RBV conjugate, CRM197-PEG-liposome loaded with RBV, and CRM197-PEG-PEI with virus-specific nucleic-acid polyplexes on the replication of a sample of relevant viral agents (including JEV and WNV (with siRNA sequences FvE J, 5'- GGA TGT GGA CTT TTC GGG A -3' (JEV nt 1287-1305); FvE JW, 5'- GGG AGC ATT GAC ACA TGT GCA -3' (JEV nt 1307-1328); and FvE W, 5'- GGC TGC GGA CTG TTT GGA A -3' (WNV nt 1287-1305) as detailed in Kumar et al., 2006, PloS Med., 3(4):e96); and 3Dz anti-JEV DNAzyme with sequence 5'-CCT CTA AGG CTA GCT ACA ACG ACT CTA GT -3' (JEV nt 10749-10763 and 10827-10841), as detailed in WO2006064519) and RSV (with siRNA stem-loop sequence against the NLS1 target 5'- GGC AGC AAT TCA TTG AGT ATG CTT CTC GAA ATA AGC ATA CTC AAT GAA TTG CTG CCT TTT TG -3' as detailed in Kong et al., 2007, Genet Vaccines Ther, 5:4) is determined. One-day-old confluent Vero cell monolayers, grown in 96-well microtiter plates, are infected with the respective virus at a multiplicity of infection (MOI) of 0.1 in the absence or presence of serial dilutions of the respective antiviral agents. Cultures are incubated at 37°C for 5 days, when infected, untreated cultures exhibited an obvious cytopathic effect (CPE). For each condition, the supernatant from two to four wells are pooled, and then total RNA is extracted (QIAamp viral RNA mini kit). Viral RNA is quantified using one-step reverse transcription-quantitative PCR (RT-qPCR). Each of the compounds cause a concentration-dependent inhibition of synthesis of the tested viral agents. The targeted RBV compounds prove to be the most potent, and RBV is the least potent compound (for instance, for YFV the EC50 for inhibition of RNA synthesis [EC50 RNA] of ribavirin is 12.3 ± 5.6 µg/ml).

### Example 7

### Antiviral activity of targeted antiviral agents in vivo

Receptor-specific antiviral activity is determined in a hamster model for human flavivirus infections representing acute encephalitis, a poliomyelitis-like syndrome and neurological sequelae (Leyssen et al., 2003, Brain Pathol., 13:279-290), using MODV. In this model it is observed that the CRM197-RBV conjugate and the CRM197-PEG-liposomes with RBV, significantly reduced morbidity and neurological sequelae. Even though glucocorticoids and interferon's were clinically ineffective for treating Japanese encephalitis (Hoke et al., 1992, J Infect Dis., 165:631-637, and Solomon et al., 2003, Lancet, 361:821-826), the co-medication of dexamethasone and interferon alfa-2a, either alone or both, is observed to further reduce morbidity and neurological sequelae in combination with treatment of the CRM197-RBV conjugate and the CRM197-PEG-liposomes with RBV.

## Claims

1. A conjugate that is obtainable by reacting distearoylphosphatidylethanolamine-polyethylene glycol-maleimide (DSPE-PEG-MAL) with reduced glutathione.

2. A conjugate according to claim 1, wherein the DSPE-PEG-MAL has a molecular weight of about 3400 Daltons.

3. A liposome comprising a conjugate according to claim 1 or 2.

4. A liposome according to claim 3 containing a drug.

5. A liposome according to claim 4, wherein the drug is an antiviral drug.

6. A liposome according to claim 5, wherein the antiviral drug is ribavirin.

7. A method for synthesizing a conjugate according to claim 1 or 2, the method comprising the step of reacting DSPE-PEG-MAL with reduced glutathione.

8. A method for preparing a liposome comprising a conjugate according to claim 1 or 2, the method comprising the step of coupling a conjugate according to claim 1 or 2 to a liposome

9. A conjugate according to claim 1 or 2, for delivery drugs across a blood-tissue barrier.

10. Use of a conjugate according to claim 1 or 2, for the manufacture of a medicament for carrying drugs across a blood-tissue barrier.

11. A conjugate according to claim 9 or a use according to claim 10 for carrying drugs across the blood-brain barrier.

## Patentansprüche

1. Konjugation, welche durch Reagieren von Distearoylphosphatidylethanolamin-Polyethylenglycol-Maleimid (DSPE-PEG-MAL) mit reduziertem Glutathion erhältlich ist.

2. Konjugation nach Anspruch 1, wobei das DSPE-PEG-MAL ein molekulares Gewicht von etwa 3400 Dalton aufweist.

3. Liposom, welches eine Konjugation nach Anspruch 1 oder 2 umfasst.

4. Liposom nach Anspruch 3, welches einen Wirkstoff umfasst.

5. Liposom nach Anspruch 4, wobei der Wirkstoff ein antiviraler Wirkstoff ist.

6. Liposom nach Anspruch 5, wobei der antivirale Wirkstoff Ribavirin ist.

7. Verfahren zum Synthetisieren einer Konjugation nach Anspruch 1 oder 2, wobei das Verfahren den Schritt eines Reagierens von DSPE-PEG-MAL mit reduziertem Glutathion umfasst.

8. Verfahren zum Synthetisieren eines Liposoms, welches eine Konjugation nach Anspruch 1 oder 2 umfasst, wobei das Verfahren den Schritt eines Koppelns einer Konjugation nach Anspruch 1 oder 2 mit einem Liposom umfasst.

9. Konjugation nach Anspruch 1 oder 2, zur Beförderung von Wirkstoffen über eine Blut-Gewebe-Schranke.

10. Verwendung einer Konjugation nach Anspruch 1 oder 2, für die Herstellung eines Medikaments zum Übertragen von Wirkstoffen über eine Blut-Gewebe-Schranke.

11. Konjugation nach Anspruch 9 oder Verwendung nach Anspruch 10 zum Übertragen von Wirkstoffen über die Blut-Gehirn-Schranke.

## Revendications

1. Conjugué susceptible d'être obtenu par réaction du distearoylphosphatidylethanolamine-polyethylene glycol-maleimide (DSPE-PEG-MAL) avec du glutathion réduit.

2. Conjugué selon la revendication 1, dans lequel le DSPE-PEG-MAL a un poids moléculaire d'environ 3400 Daltons.

3. Liposome comprenant un conjugué selon la revendication 1 ou 2.

4. Liposome selon la revendication 3, contenant un médicament.

5. Liposome selon la revendication 4, dans lequel le médicament est un médicament antiviral.

6. Liposome selon la revendication 5, dans lequel le médicament antiviral est de la ribavirine.

7. Méthode pour la synthèse d'un conjugué selon la revendication 1 ou 2, la méthode comprenant une étape de réaction du DSPE-PEG-MAL avec du glutathion réduit.

8. Méthode pour la préparation d'un liposome comprenant un conjugué selon la revendication 1 ou 2, la méthode comprenant une étape de couplage d'un conjugué selon la revendication 1 ou 2, à un liposome.

9. Conjugué selon la revendication 1 ou 2, pour la libération de médicaments au delà de la barrière sang-tissu.

10. Utilisation d'un conjugué selon la revendication 1 ou 2, pour la préparation d'un médicament, pour le transport de médicaments au delà de la barrière sang-tissu.

11. Conjugué selon la revendication 9 ou utilisation selon la revendication 10, pour la transport de médicaments au delà de la barrière hémato-encéphalique.
